Europäisches Patentamt

European Patent Office

Office européen des brevets

(11) Publication number: **0 253 455**
A1

## (12) EUROPEAN PATENT APPLICATION

(21) Application number: 87201379.2

(22) Date of filing: 20.07.87

(51) Int. Cl.⁴: **C 12 N 15/00**, C 12 N 1/20,
C 12 N 1/16, C 12 P 21/00,
A 61 K 37/02, C 07 K 13/00,
A 61 K 39/395, G 01 N 33/68,
G 01 N 33/577
// (C12N1/20,C12R1:125),
(C12N1/20,C12R1:10),
(C12N1/20,C12R1:07)

(30) Priority: 18.07.86 GB 8617594
23.12.86 GB 8630699

(43) Date of publication of application: 20.01.88
Bulletin 88/3

(84) Designated Contracting States: AT BE CH DE ES FR GB
GR IT LI LU NL SE

(71) Applicant: GIST-BROCADES N.V., Wateringseweg 1,
NL-2611 XT Delft (NL)

(72) Inventor: Van Ooyen, Albert Johannes Joseph, Laan van
Heidenburg 23, NL-2271 AR Voorburg (NL)
Inventor: Andreoli, Peter Michael, Molenlaan 111,
NL-3055 EJ Rotterdam (NL)
Inventor: Van Mourik, Jan Aart, Maraboestraat 1,
NL-1171 SX Badhoevedorp (NL)
Inventor: Pannekoek, Hans, Stommeerweg 36,
NL-1431 EW Aalsmeer (NL)

(74) Representative: Huygens, Arthur Victor, Dr. et al, c/o
Gist-Brocades N.V. Patent & Trademarks Department
Wateringseweg 1 PO Box 1, NL-2600 MA Delft (NL)

(54) Method for the preparation of proteins with factor VIII activity by microbial host cells; expression vectors, host cells, antibodies.

(57) Microbial systems are provided for producing peptides having biological and physiological factor VIII activity, in being immunologically cross-reactive with factor VIII and physiologically active in providing for clotting activity. The protein products wary from immunologically cross-reactive fragments to proteins comprising the two Factor VIII subunits joined together directly or by a bridge. High yields of the products are obtained in isolatable and biologically active form.

S-2428

13 juli 1987

METHOD FOR THE PREPARATION OF PROTEINS WITH FACTOR VIII
ACTIVITY BY MICROBIAL HOST CELLS; EXPRESSION VECTORS, HOST
CELLS, ANTIBODIES.

INTRODUCTION

Technical Field

The invention relates to a microbiological process
for the preparation of proteins with factor VIII activity.

Background

Factor VIII (also referred to as factor VIIIc) is a
medically important protein since it is an essential component
of the blood coagulation mechanism. It occurs in the blood
plasma of normal healthy individuals at a concentration of
about 100 ng/ml. Factor VIII deficiency results in a bleeding
disorder called hemophilia A, which occurs mainly in males
because the factor VIII gene is located on the X-chromosome.
The disease is the major inherited bleeding disorder,
occurring in about 0.01% of the male population.

Hemophilia A can be treated by administering factor
VIII containing blood plasma obtained from healthy people.
This treatment, however, has several disadvantages. Firstly,
the supply of factor VIII is limited and very expensive
because the concentration in the blood of donors is very low.
Secondly, the yields of current plasma fractionation methods
are low. In fact, there is a shortage of factor VIII. Thirdly,
every hemophilia A patient receives samples collected from a
large number of donors which leads to a high risk of acquiring
infectious diseases, such as those caused by hepatitis non-A,
non-B, hepatitis B or AIDS viruses present in donor blood.
Another problem is the development of antibodies against
factor VIII preparations in so-called inhibitor patients.

These problems have provoked an intense search for alternative methods for the production of factor VIII, which avoid the use of donor blood, viz. by recombinant DNA techniques and three groups have reported molecular cloning of factor VIII cDNA obtained from mRNA.

International Patent Application WO 85/01961 (Genetics Institute) describes the preparation of cDNA coding for human factor VIII or for subunits with factor VIII activity. The cDNA is cloned, whereafter a mammalian COS-7 cell line is transformed with a vector containing said cDNA.

European patent application EP-A-160457 (Genentech) describes a method of transfecting a mammalian (Baby Hamster Kidney) cell line with a vector containing DNA coding for functional human factor VIII.

European Patent Appliation EP-A-150735 (Chiron) describes a method for the preparation of the factor VIII protein and of fragments thereof with factor VIII activity. The vector containing DNA coding for said protein came to expression in mammalian cell lines, particularly in COS cells (Truett et al., 1985).

Although the above-mentioned publications state that microorganisms could be used as the host cells in an expression system, no experimental results describing this are presented, leading to the inevitable conclusion that the reduction to practice of this procedure has encountered unexpected difficulties at the experimental level.

The cited literature shows that proteins with factor VIII activity can be prepared by recombinant DNA techniques, but only in mammalian cell lines. However, the concentration of factor VIII produced by the cell lines was very low; in fact, too low for commercial production. An additional disadvantage of the use of these cell lines is that they may be derived from tumor material and thus may contain substances which are hazardous to health.

It was known moreover, e.g. from European applications EP-A-150735 (Chiron) and EP-A-123945 (Scripps) and from Brinkhous et al., (1985) that factor VIII activity should be attributed not only to the protein factor VIII itself, but also to certain proteolytic cleavage products of it. Thrombin is a proteolytic enzyme which, when present in the blood, partially digests factor VIII into subunit polypeptides two of which have molecular weights of 92 kDa and 80 kDa. It is believed that when a complex of these two subunit proteins or parts thereof (Faye et al., 1986, Eaton et al., 1986) appear in the blood the factor VIII activity is enhanced.

## DEFINITIONS

Factor VIII is the protein which corrects the clotting defect known as hemophilia A. Factor VIII circulates in the blood combined with a larger protein, the von Willebrand factor (vWf), which is believed to protect the sensitive factor VIII against early degradation.

Factor VIII (human) is characterized by the amino acid sequence as described in Table I. The numbering of the sequence starts with Ala(1), the first amino acid after the signal sequence of 19 amino acids. The last amino acid is Tyr(2332). This numbering is used throughout the specification.

In the literature, factor VIII is frequently reported as the combination of factor VIIIc, which for the purposes of this invention will be referred to as factor VIII and factor VIIIvWf, the von Willebrand factor. In this invention, factor VIII is the combination of a 80 kDa subunit and a 92 kDa subunit which are derived from a much larger precursor protein, which will be referred to as the factor VIII precursor. In the precursor, the 80 kDa subunit and the 92 kDa subunit are joined by a bridge, which is highly glycosylated.

Fragments of factor VIII will normally include at least a portion of one of the two subunits and may include, but will normally not include, portions of the precursor protein outside of the 80 kDa and 92 kDa subunits.

In referring to a peptide having factor VIII biological activity, immunological cross reactivity with one of the subunit proteins will be sufficient to fulfill this requirement.

"Pharmaceutical preparations with factor VIII activity" means any preparation which can correct the clotting defect associated with factor VIII deficiency. The correction of the clotting defect may be effected by direct action of the recombinantly prepared protein, by providing one or both of the 80 kDa and 92 kDa subunits or functionally equivalent analogs thereof, or by neutralizing antibodies against factor VIII protein present in inhibitor patients so that a subsequently administered factor VIII protein, analog or functionally equivalent fragment is able to display its full activity.

For convenience, the term "protein" will be used to include proteins and smaller polypeptides.

## SUMMARY OF THE INVENTION

The present invention provides novel constructs for producing proteins having factor VIII biological and physiological activity in isolatable form. The production processes employ microbial hosts, particularly yeast or bacteria, transformed with an expression cassette comprising a transcriptional and translational initiation region which efficiently functions in the host and an open reading frame encoding the protein of interest. Preferred constructs encode a signal leader for secretion of the encoded product, as well as a fused protein which includes a bridge between the two subunits.

Expression may be achieved employing extrachromosomal elements or with integration of the expression cassette into the host genome.

BRIFF DESCRIPTION OF THE DRAWINGS

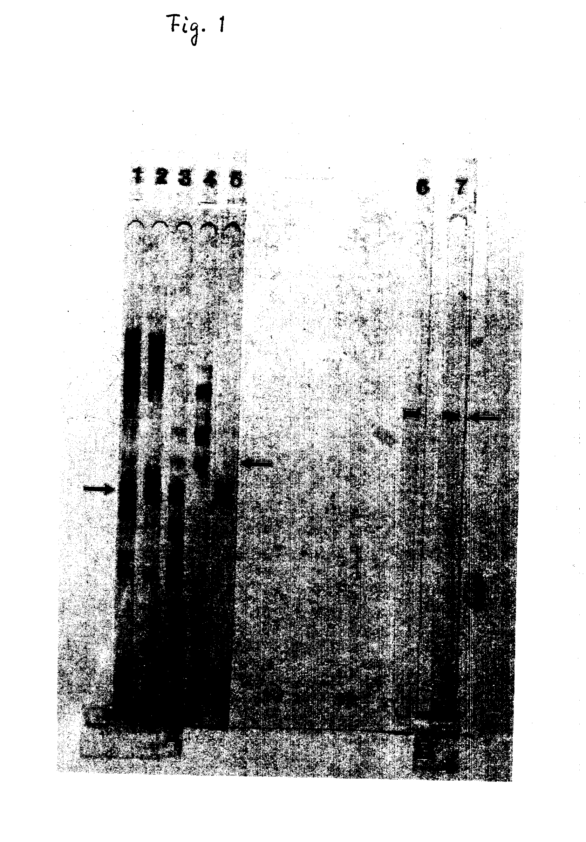

FIGURE 1 : ANALYSIS OF PURIFIED FACTOR VIII-VON WILLEBRAND FACTOR COMPLEX (PANEL A) AND PURIFIED FACTOR VIII POLYPEPTIDES DEVOID OF VON WILLEBRAND FACTOR (PANEL B) BY IMMUNOBLOTTING.

Factor VIII containing samples were subjected to SDS-polyacrylamide gel electrophoresis. After separation, proteins were transferred to nitrocellulose sheets and, after appropriate washing steps, incubated with antibodies to factor VIII polypeptides.

A: Lane 1: polyclonal antibody RH 63271
   Lane 2: polyclonal antibody RH 63272
   Lane 3: polyclonal antibody RH 63275
   Lane 4: monoclonal antibody CLB-CAg 9
   Lane 5: monoclonal antibody CLB-CAg 65
B: Lane 6: monoclonal antibody CLB-CAg 21
   Lane 7: monoclonal antibody CLB-CAg 62.

Panel A and B are run for different times. Arrows indicate positions of 80 kDa and 92 kDa proteins.

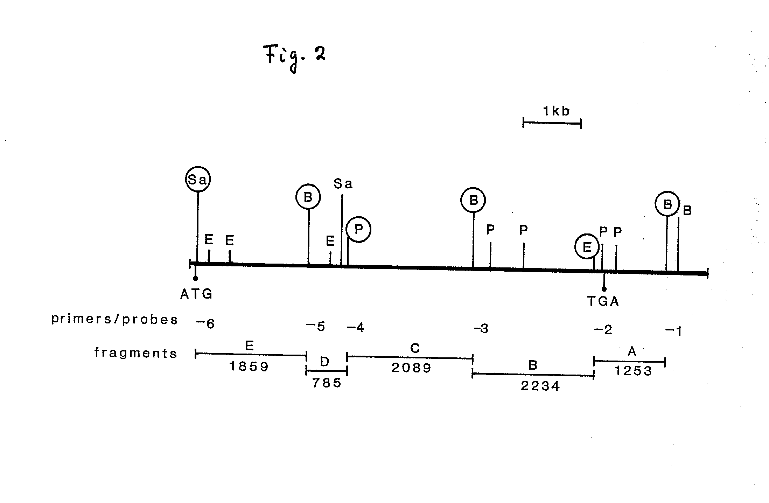

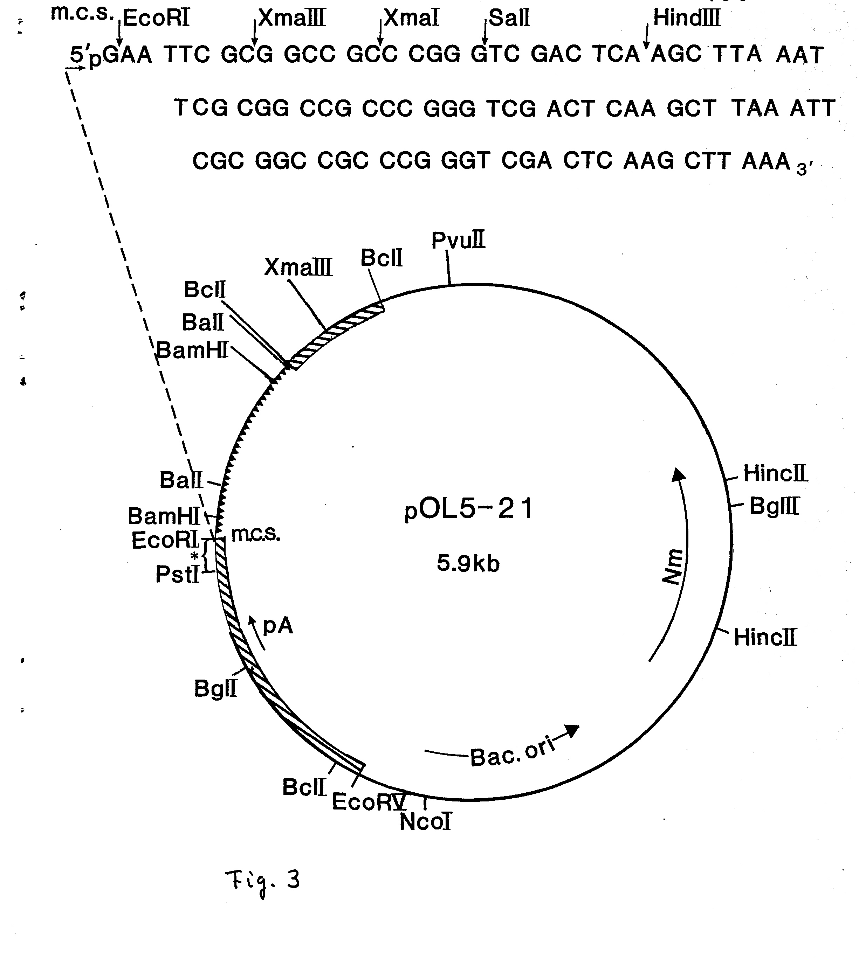

FIGURE 2 : STRATEGY FOR SYNTHESIS OF A cDNA CLONE TO FACTOR VIII mRNA.

Specific primers, complementary to the indicated parts of factor VIII mRNA were used for priming cDNA synthesis. A second oligonucleotide which can also be used for priming of the next cDNA fragment, is used for detection of correct cDNA clones. Nucleotide sequences of primers used are as follows:

1: 3'GGAAGGTAGACGGACTGGGGAAG 5'

2: 3'GGACTCCTACCTCCAAGACCCG 5'

3: 3'CCTTTAGGGTTCTCTTCAGTGG 5'

4: 3'GGTAGTCTGTTAAACCGTCGTCC 5'

5: 3'CGTACTCCACCGTATGACCATG 5'

6: 3'GCCACTCGACGGACACCTGCG 5'


Resulting cDNA fragments are subcloned with restriction enzyme sites present in circles: E: EcoRI; B: BamHI; P: PstI; Sa: SacI. Numbers below fragments refer to the size of the corresponding fragment in base pairs. Plasmids containing fragments A,B,C,D and E are called pCLB81, pCLB82, pCLB83, pCLB84 and pCLB85, respectively.


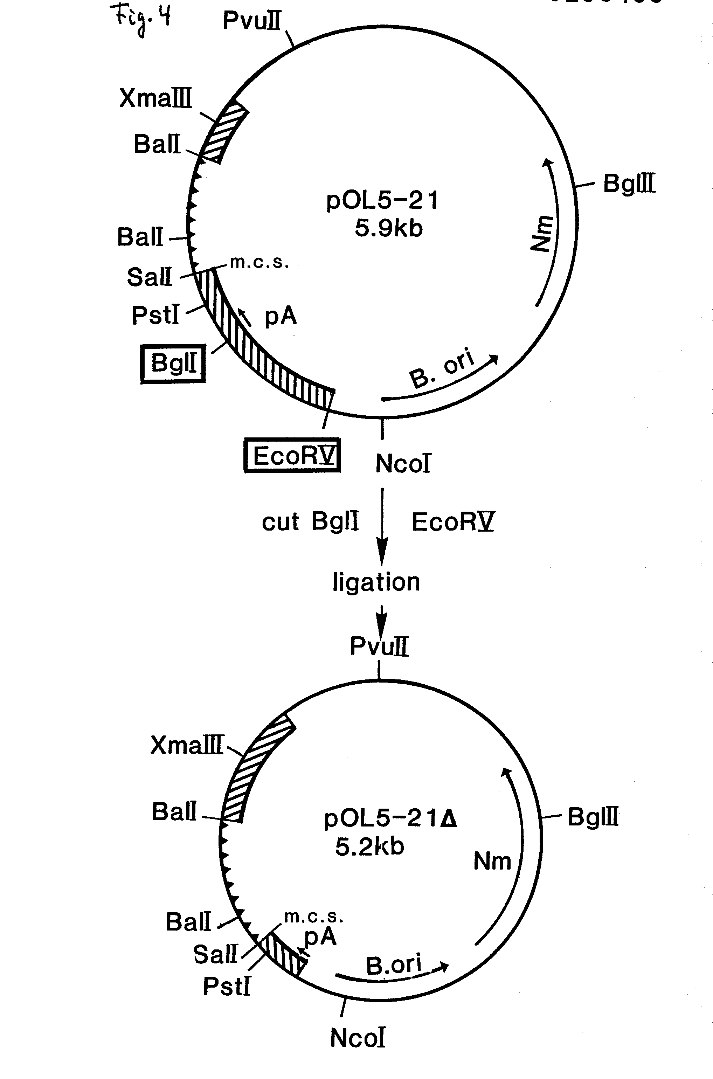

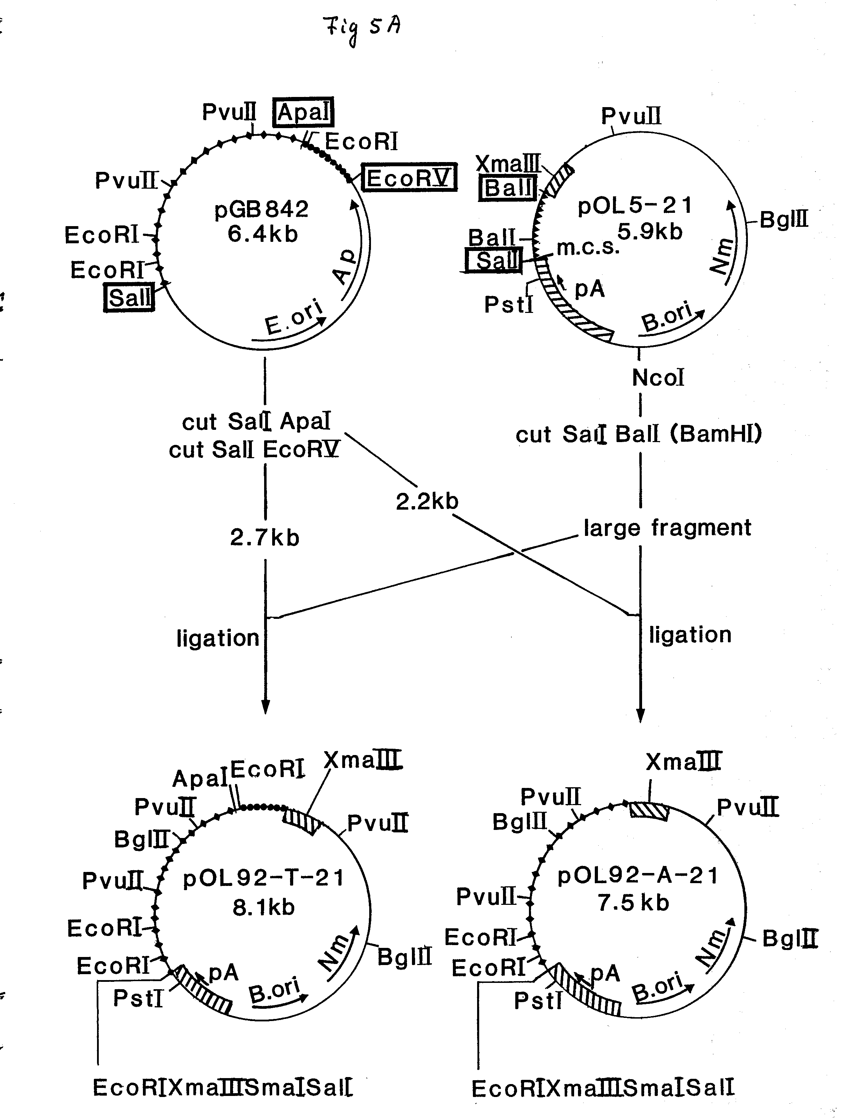

FIGURE 3 :   A MAP OF pOL5-21, AS AN EXAMPLE OF A
             pOL5-n TYPE PLASMID


Plasmids  of the pOL5-n type consist of:

▨▨▨▨▨▨ : B. licheniformis T5 DNA sequences;

▲▲▲▲▲▲▲ : Chymosin-encoding sequences;

pA
→ : Alpha-amylase promoter, ribosome binding site, ATG initiation codon and the DNA encoding 29 amino acids of the alpha-amylase signal sequences;

✱ { : DNA encoding n amino acids of the mature alpha-amylase protein (in the case of pOL5-21, 21 amino acids).

————— : Vector pUB110 with a Bacillus origin of replication (Bac.ori) and the gene for neomycin resistance (Nm).

m.c.s.   : multicloning sites.


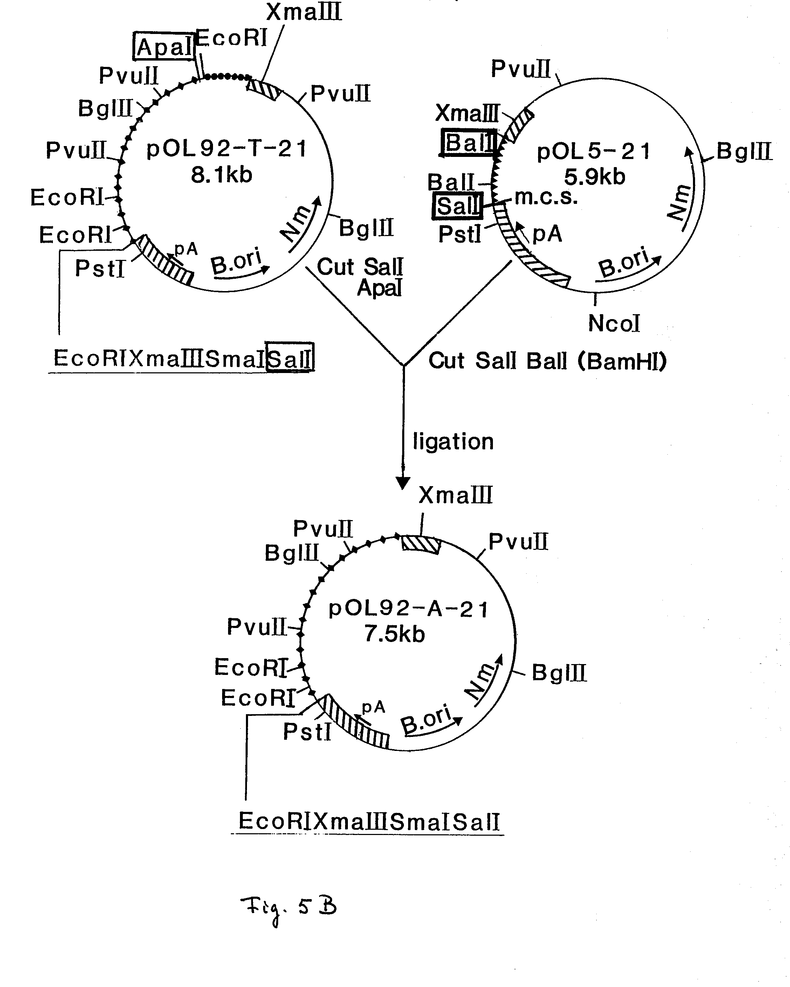

FIGURE 4 :   CONSTRUCTION OF pOL5-DELTA PLASMIDS

In plasmid pOL5-21delta the B. licheniformis DNA sequences upstream the alpha-amylase gene have been deleted. Symbols as in Figure 3.

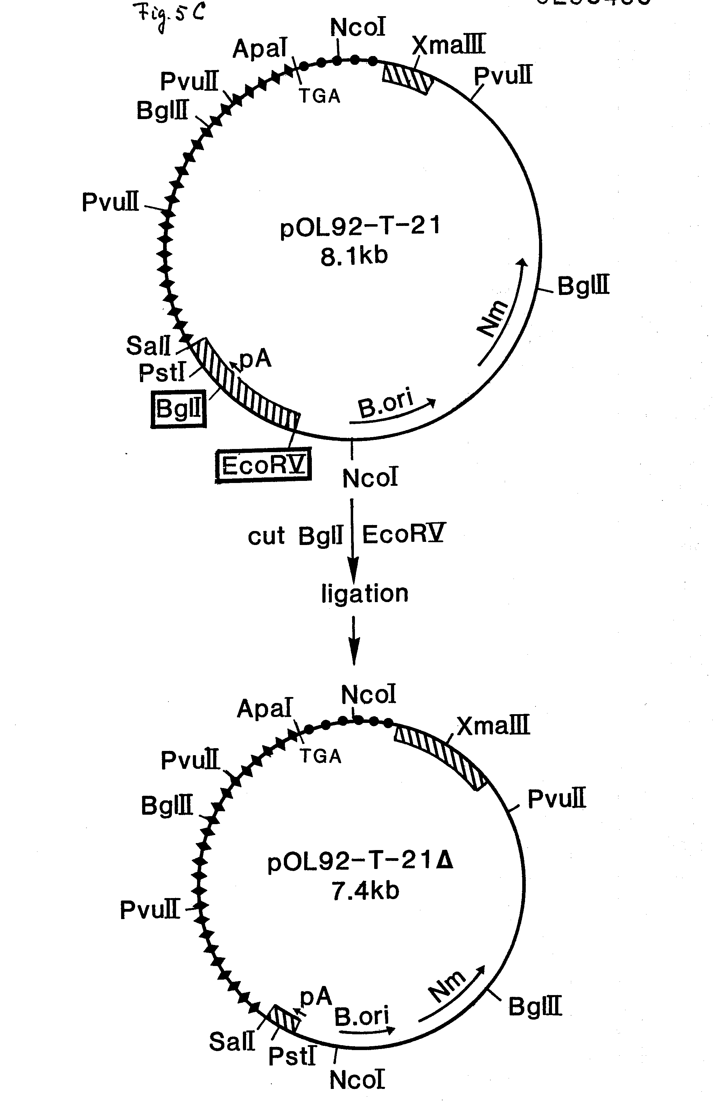

FIGURE 5A AND 5B  :  CONSTRUCTION OF pOL92 PLASMIDS.

5A         : pOL92-T-n plasmids are derived partly from pOL5-n
             (Figure 3) and pGB842 which is the SalI-EcoRV
             fragment containing the 92 kDa subunit DNA from
             pOL92-A-n plasmids are derived partly from pOL5-n
             and the SalI-ApaI fragment containing the 92 kDa
             subunit DNA from pGB842.


Symbols used are:
 - B. ori: Bacillus origin of replication.
 - E. ori: E. coli origin of replication
 - Ap: the pAT153 gene encoding ampicilline resistance
 - Nm: the pUB110 gene encoding neomycin resistance
 - pA: the gene expression and signal sequences of the
        B. licheniformis alpha-amylase gene
 - m.c.s.: multicloning sites

▨▨▨▨▨▨ : B. licheniformis T5 DNA sequences
◆◆◆◆◆◆◆ : Factor VIII 92 kDa-encoding cDNA insert
●●●●●●●● :Kluyveromyces lactis lactase terminator(T)
▲▲▲▲▲▲▲ : Chymosin DNA


5B         : Alternatively, pOL92-A-n plasmids can be
             constructed from pOL92-T-n plasmids and pOL-5-n as
             depicted for pOL92-A-21.


FIGURE 5C: CONSTRUCTION OF pOL92-DELTA PLASMIDS

        In plasmid pOL92-T-21delta the B. licheniformis DNA
sequences upstream the alpha-amylase gene have been deleted.
Symbols used as in Figure 5A,B.

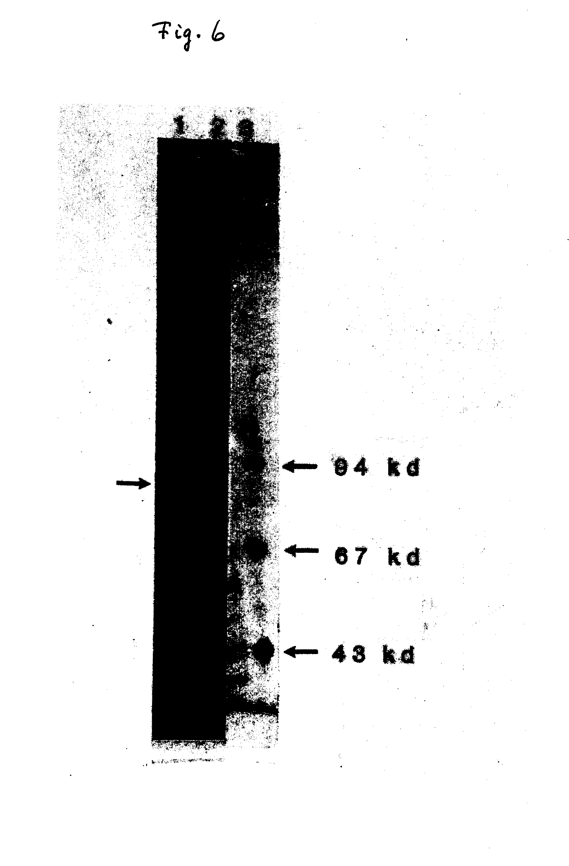

FIGURE 6 : POLYACRYLAMIDE GEL ANALYSIS OF CELL LYSATES
OF B. SUBTILIS TRANSFORMANTS.

The samples were analysed on a 6% SDS-polyacrylamide gel and
stained with Coomassie brillant blue.

Lane 1: cell lysate from Bacillus subtilis transformed with
pOL92-T-21 Lane 2: cell lysate from Bacillus subtilis
transformed with pOL-5-21
Lane 3: molecular weight marker

The arrow indicates the protein band of 88 kDa in pOL92-T-21.

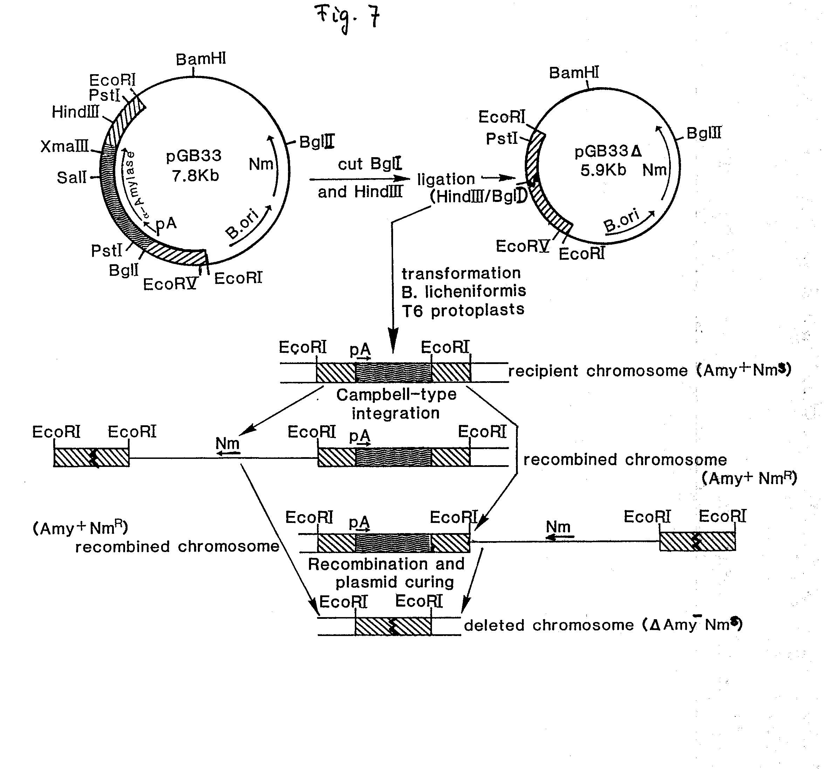

FIGURE 7: STRATEGY FOR DELETION OF THE B. LICHENIFORMIS ALPHA-
AMYLASE STRUCTURAL GENE

Plasmid pGB33 contains a 3.3kb EcoRI fragment
harbouring the B. licheniformis alpha-amylase gene and
flanking DNA sequences.
In plasmid pGB33delta the whole alpha-amylase gene
has been deleted. Symbols used are:

|  |  |
|---|---|
|  | : alpha-amylase encoding sequences |
| NmS | : neomycin sensitive phenotype |
| NmR | : neomycin resistant phenotype |
| Amy+ | : alpha-amylase proficient phenotype |
| Amy- | : alpha-amylase deficient phenotype |

Other symbols as in Figure 3.

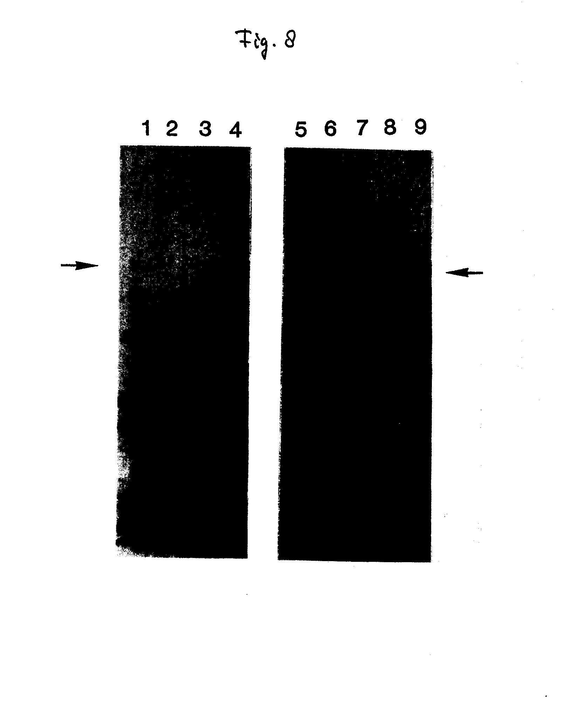

FIGURE 8: POLYACRYLAMIDE GEL ANALYSIS OF CELL LYSATES OF
B. LICHENIFORMIS TRANSFORMANTS.

lanes 1-4 contain extracellular proteins and lanes 6-9
intracellular proteins. Staining with Coomassie brillant blue.

lane 1: <u>Bacillus licheniformis</u> T5-16 transformed with pOL92-A-21

lane 2: Strain T5-16, control

lane 3: Strain T5-16, control

lane 4: Strain T5-16, transformed with pOL92-T-21

lane 5: Molecular weight markers

lane 6: <u>Bacillus licheniformis</u> T5-16 transformed with pOL92-A-21

lane 7: Strain T5-16, control

lane 8: Strain T5-16, control

lane 9: Strain T5-16, transformed with pOL92-T-21


The arrow indicates the 92 kDa subunit protein.


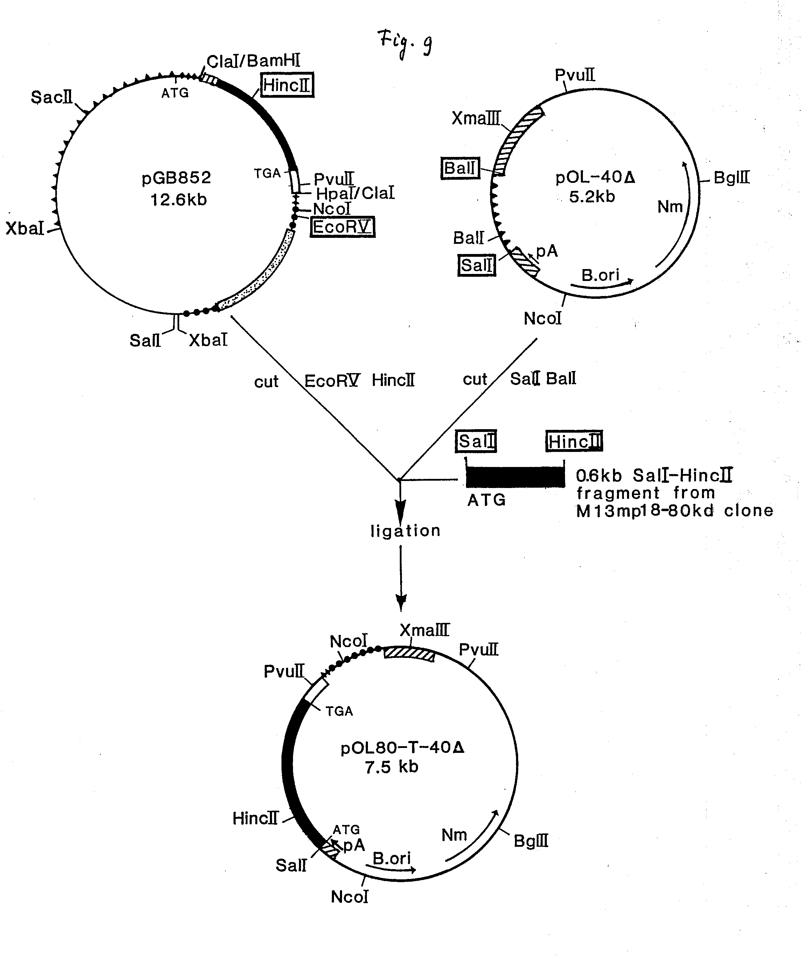

FIGURE  9: CONSTRUCTION OF pOL80 PLASMIDS

Plasmid pOL80-T-40delta has been constructed from pGB852, M13mp18-80 kDa and pOL5-40delta as depicted.


Symbols used are:

◆━◆━◆━◆━◆ : lactase-encoding DNA

●━●━●━●━● : lactase terminator

▲━▲━▲━▲━▲ : lactase promoter

⬛⬛⬛⬛ : G418 resistance marker

⬛⬛⬛⬛ : remaining part from factor VIII B-domain

⬛⬛⬛⬛ : 3' non-coding region of factor VIII cDNA

⬛⬛⬛⬛ : 80 kDa protein encoding DNA

Other symbols as in Figure 3.


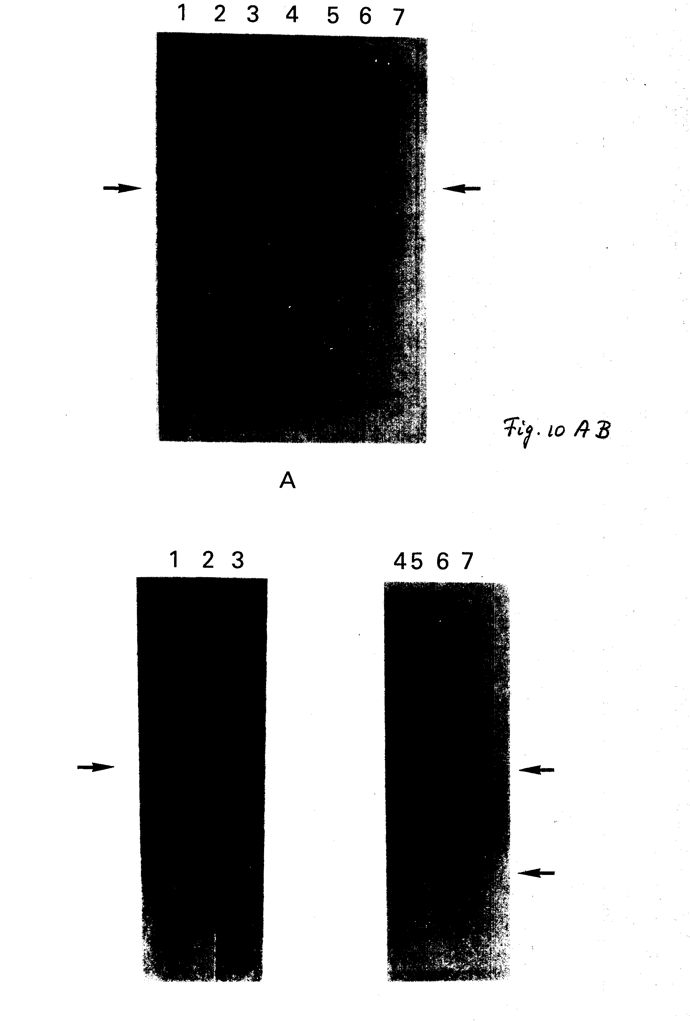

FIGURE 10: DETECTION OF FACTOR VIII POLYPEPTIDES PRODUCED  BY
           TRANSFORMED <u>BACILLUS</u> STRAINS WITH SPECIFIC MONO-
           AND POLYCLONAL ANTIBODIES


A. Immunoblot detection of the 92 kDa subunit protein with monoclonal antibody CLB-CAg9 and goat anti-rabbit alkaline phosphatase.

0253455

Lanes 1-5 contain proteins from culture supernatants. Lane 1 and 2: B. licheniformis T9 transformed with pOL92-T-21delta and pOL92-T-2delta, respectively. Lane 3: B. licheniformis T9 strain, control. Lanes 4 and 5: B. licheniformis T9 transformed with pOL5-21delta and pOL5-2delta, respectively. Lane 6 shows the pattern obtained with a factor VIII cryoprecipitate. Lane 7: high molecular weight blot protein markers from BRL (Bethesda). Arrow at left indicates the position of recombinant 92 kDa (r92 kDa) subunit. Arrow at right indicates the position of plasma-derived 92 kDa (pd92 kDa) subunit.

B. Immunoblot detection of the 80 kDa subunit protein with polyclonal antibody RH63272 and goat anti-rabbit alkaline phosphatase.

Lanes 3-7 contain proteins from culture supernatants. Lane 1: high molecular weight blot protein markers from BRL (Bethesda). Lane 2 shows the pattern obtained with a factor VIII cryoprecipitate. Lane 3: B. licheniformis T9 transformed with pOL5-40delta vector. Lanes 4-7: B. licheniformis T9 transformed with pOL80-T-40delta, pOL80-T-21delta, pOL80-T-10delta and pOL80-T-2delta, respectively. Arrow at left indicates the position of plasma-derived 80 kDa (pd80 kDa) subunit doublet. Arrows at right indicate the position of recombinant 80 kDa (r80 kDa) subunit and a cleavage product of r80 kDa.

C. Immunoblot detection of the 80 kDa subunit protein with polyclonal antibody RH 63272 and goat anti-rabbit alkaline phosphatase.

Lanes 2-7 contain proteins from cell lysates. Lane 1: shows the pattern obtained with a factor VIII cryoprecipitate. Lanes 2, 4 and 6: B. subtilis 1A40 transformed with pOL80-T-40delta, pOL80-T-21delta and pOL80-T-2delta, respectively. Lanes 3, 5 and 7: B.

subtilis 1A40 transformed with pOL5-40delta, pOL5-21delta and pOL5-2 delta, respectively. Arrow at right indicates the position of plasma derived 80 kDa (pd80 kDa) subunit doublet. Arrow at left indicates the position of recombinant 80 kDa (r80 kDa) subunit.

D.  Immunoblot detection of a 92kDa/80kDa fusion protein with polyclonal antibody RH 63272 and goat anti-rabbit alkaline phosphatase.

Lanes 1-8 contain proteins from cell lysates. Lanes 1-4: controls of B. subtilis 1A40 transformed with pPROM3-4 vector. Lanes 5-8: four independently isolated colonies of B. subtilis 1A40 transformed with pPROM92/80PB. Arrow at right indicates the position of the 92 kDa-80 kDa fusion protein.

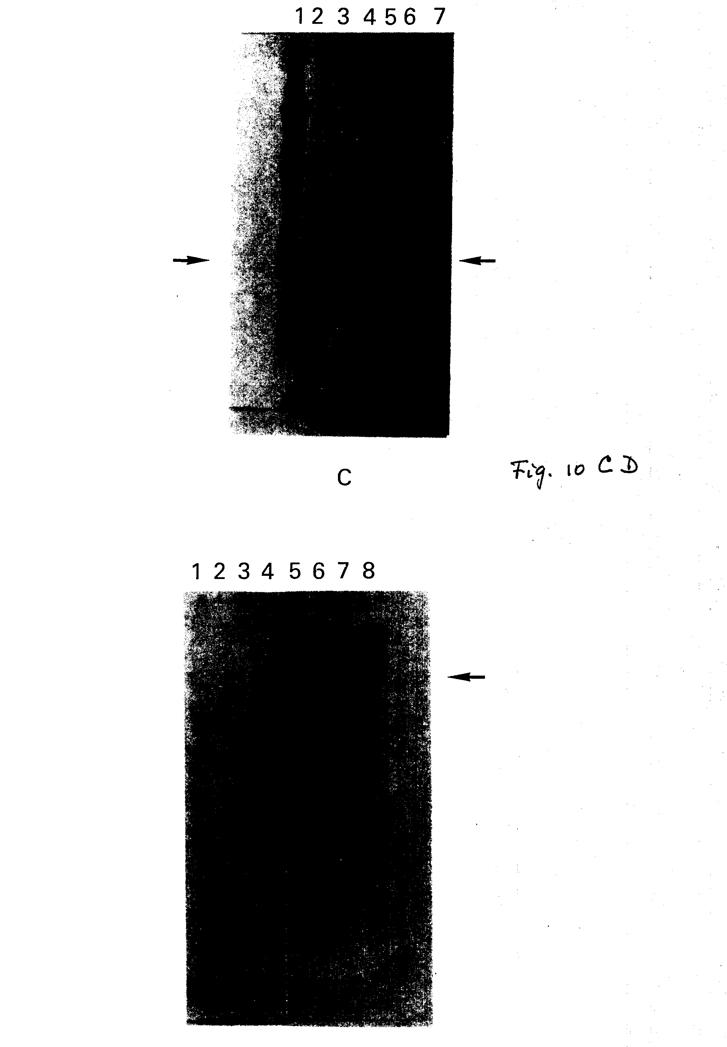

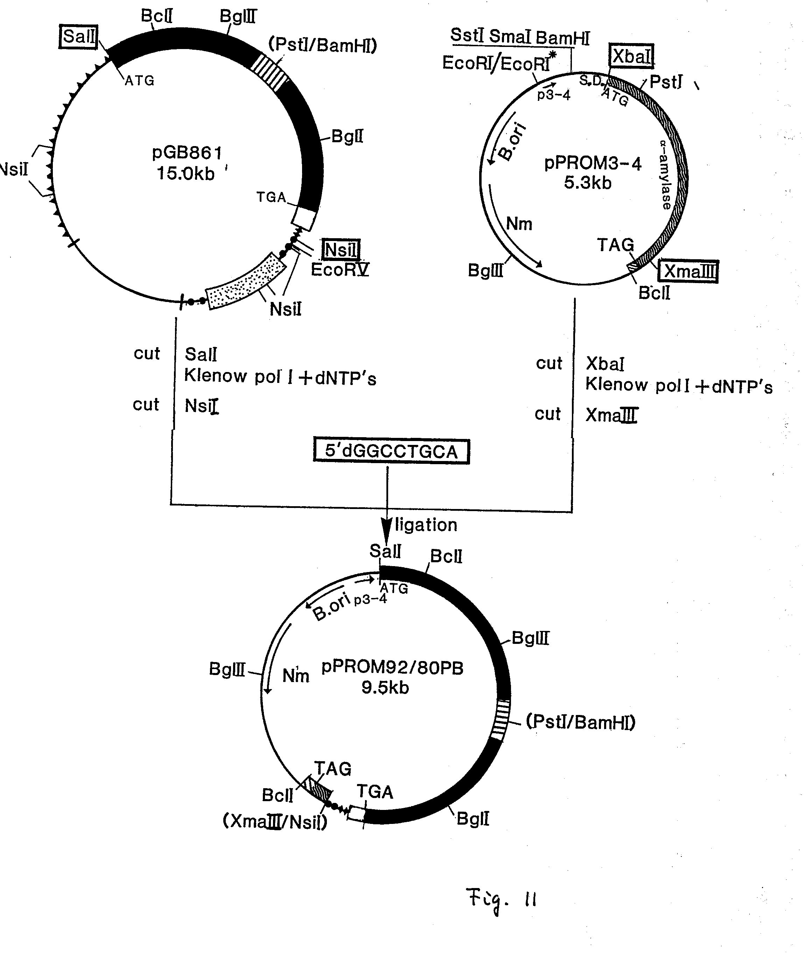

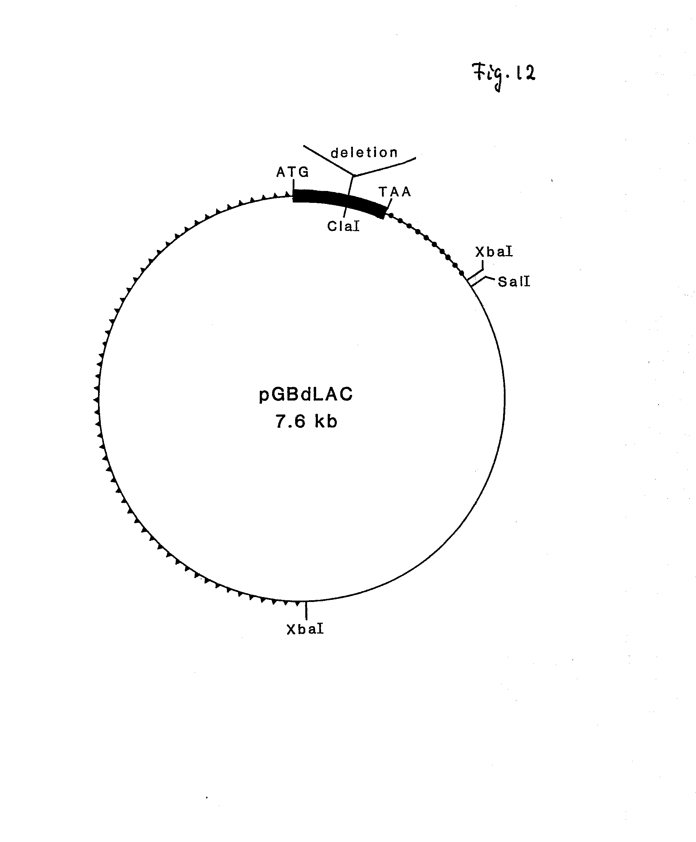

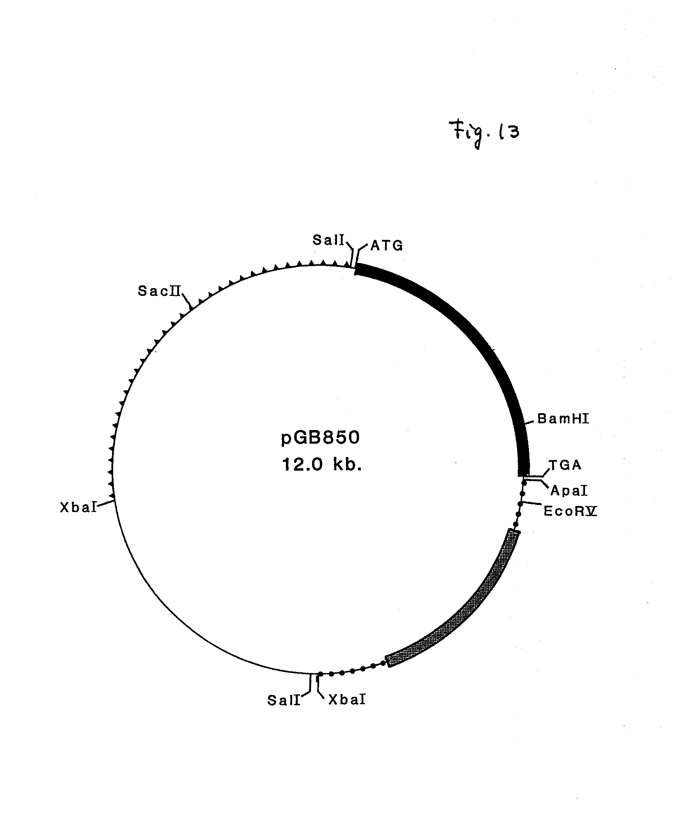

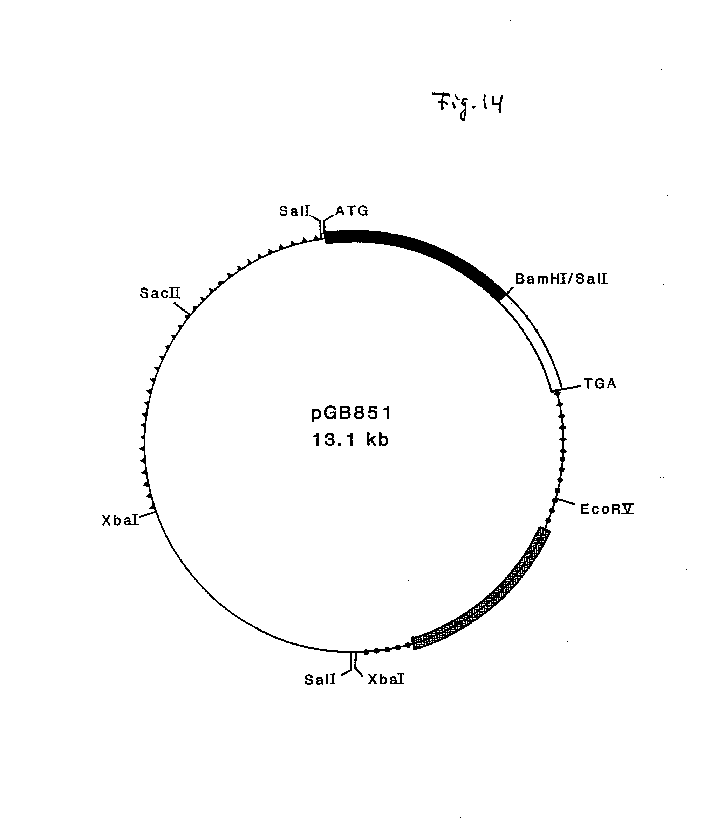

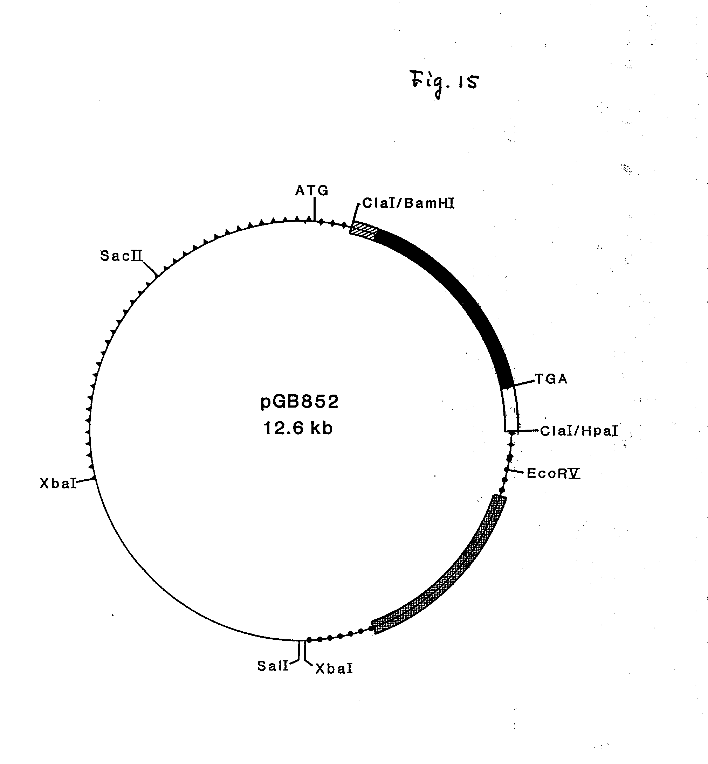

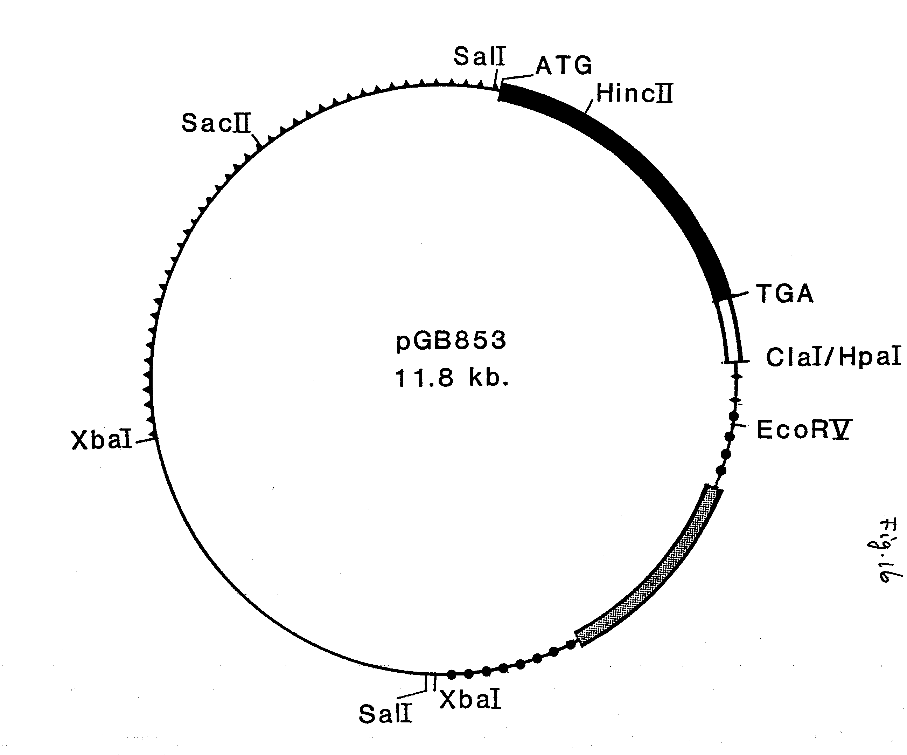

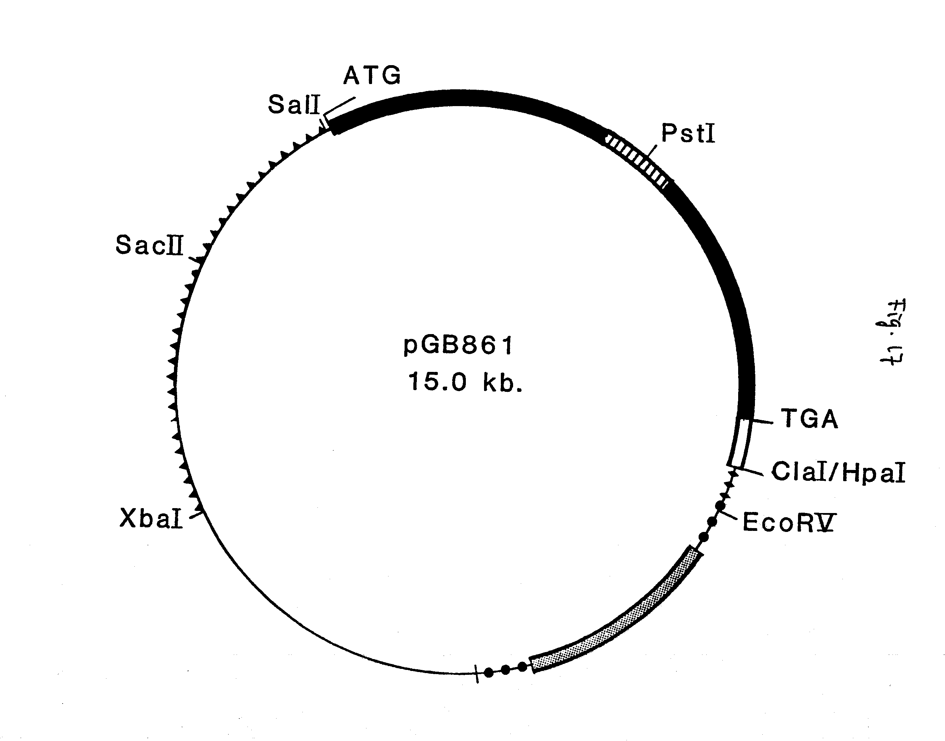

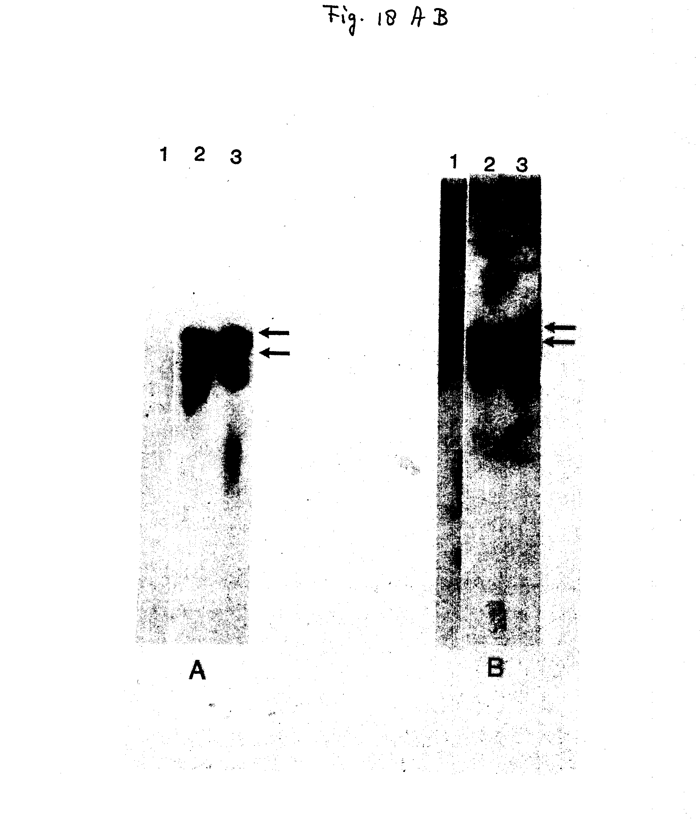

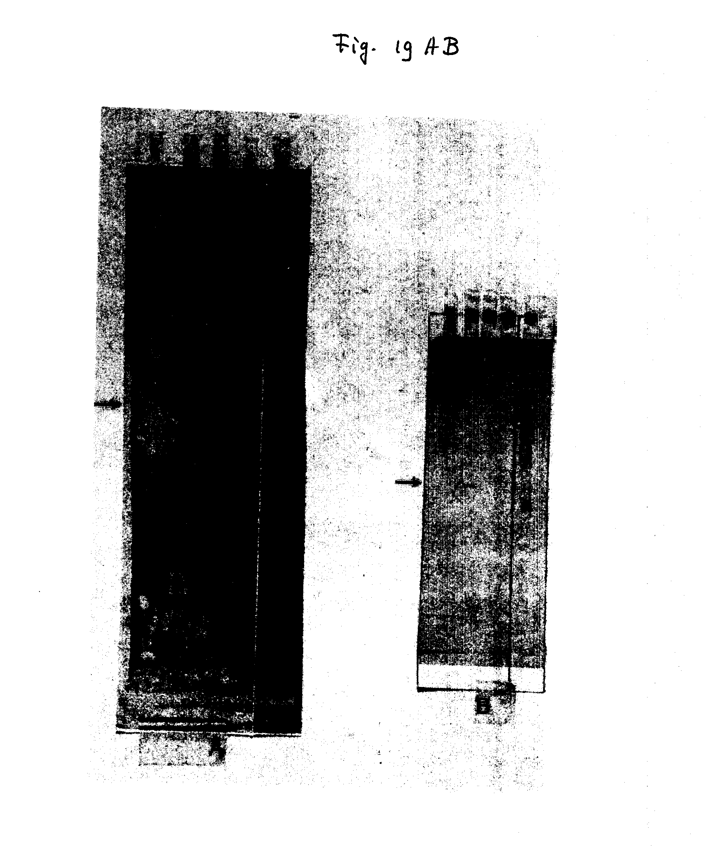

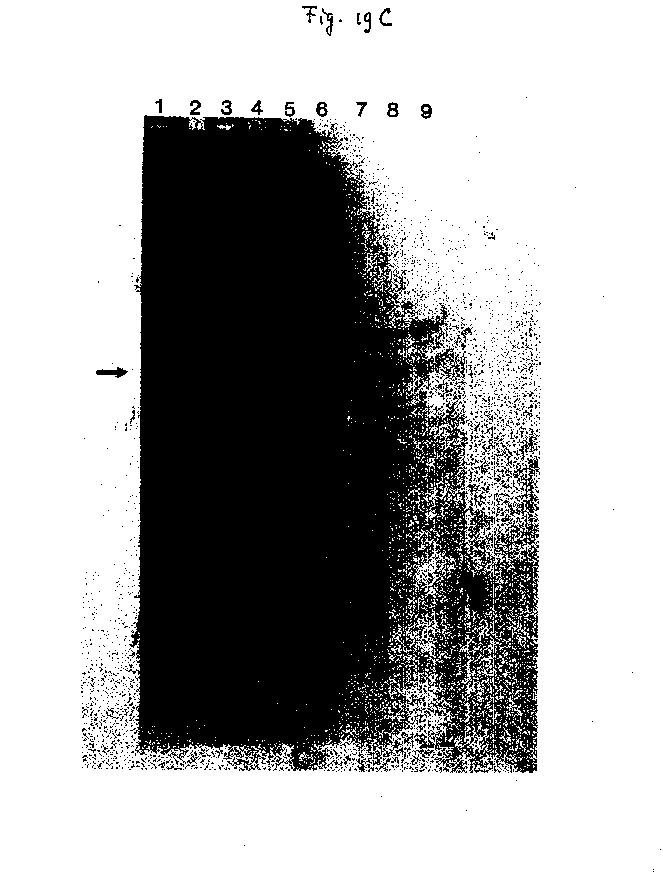

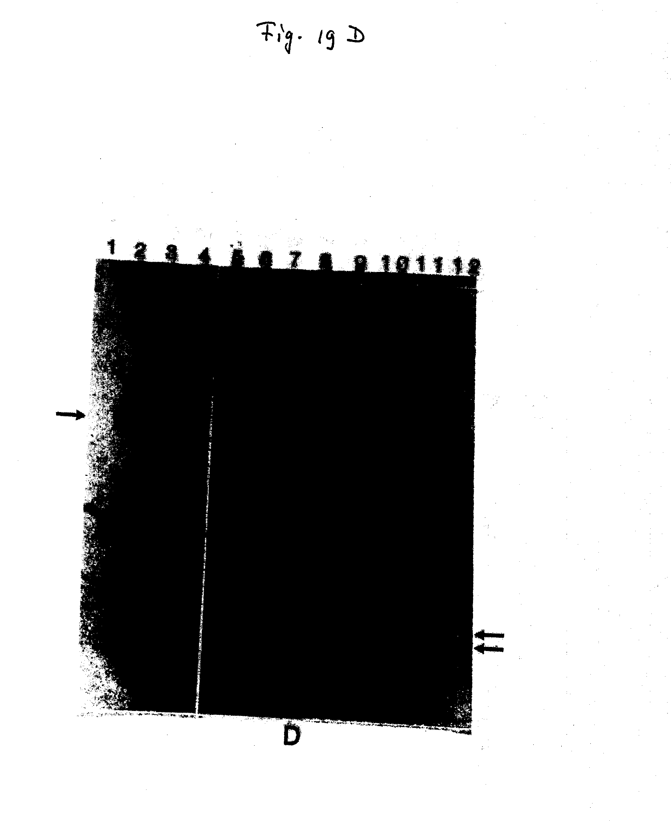

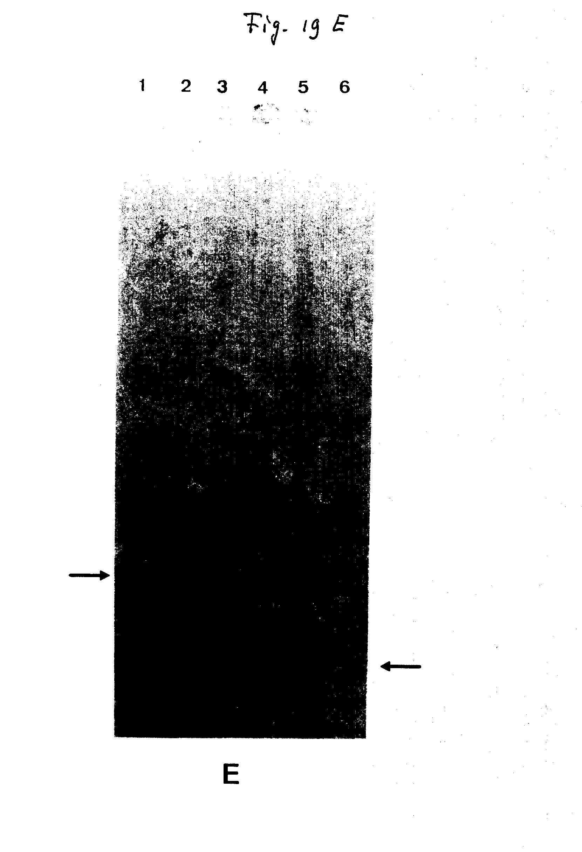

FIGURE 11: CONSTRUCTION OF pPROM92/80PB PLASMID

Plasmid pPROM92/80PB has been constructed from pGB861 and pPROM3-4 as depicted.

Symbols used are:

〜〜〜〜 : alpha-amylase encoding sequences

▬▬▬▬ : 92 kDa and 80 kDa-encoding DNA

ⅢⅢⅢⅢⅢ : remaining part from factor VIII B-domain

▨▨▨▨ : B. licheniformis T5 DNA sequences

p3-4→ : B. licheniformis 3-4 promoter

Other symbols as in Figure 9.

FIGURE 12: STRUCTURE OF pGBdLAC.

Plasmid pGBdLAC contains an XbaI fragment harbouring the K. lactis lactase gene and promoter and terminator sequences. The middle fragment between two ClaI sites has been deleted from the gene.

——————— : pUC19

⊢⊢⊢⊢⊢⊢⊢ : lactase promoter

•—•—•—•—• : lactase terminator

▬▬▬▬▬▬ : lactase gene

FIGURE 13: STRUCTURE OF pGB850.

Plasmid pGB850 contains the 92 kDa subunit-encoding DNA under control of the lactase promoter. Symbols used are:

——————— : pUC19

⊢⊢⊢⊢⊢⊢⊢ : lactase promoter

•—•—•—•—• : lactase terminator

▬▬▬▬▬▬ : 92 kDa protein encoding DNA

▭▭▭▭▭▭ : G418 resistance marker

FIGURE 14: STRUCTURE OF pGB851

Plasmid pGB851 contains the major part of the 92 kDa subunit DNA with the bovine prochymosin gene in phase at the BamHI site.

⊏▭▭▭▭⊐ prochymosin-encoding DNA

•—•—•—•—•→ lactase-encoding DNA

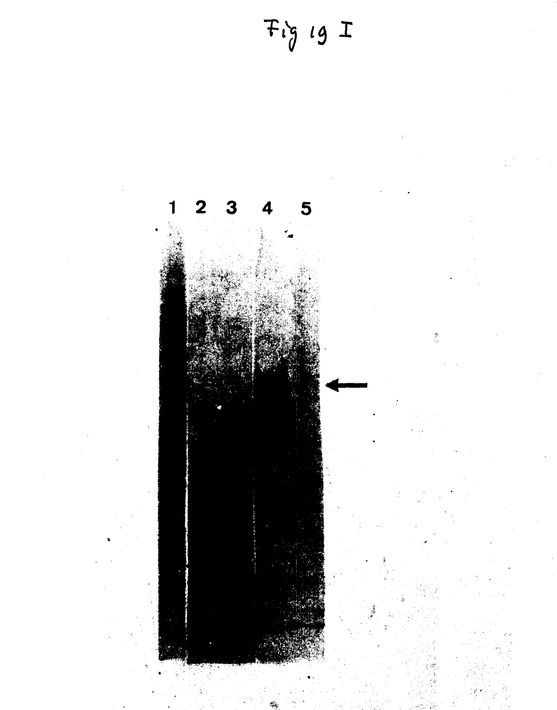

Other symbols as in Fig. 13.

FIGURE 15: STRUCTURE OF pGB852

Plasmid pGB852 contains the 80 kDa-encoding DNA fused to the lactase gene.

▬▬▬▬▬▬ : 80 kDa-encoding DNA

•—•—•—•—•→ : lactase-encoding DNA

▨▨▨▨▨▨ : part of factor VIII DNA preceding the 80 kDa-

encoding DNA.

⊏▭▭▭▭⊐ : 3' non-coding region of factor VIII cDNA

Other symbols as in Fig. 13.

FIGURE 16: STRUCTURE OF pGB853

Plasmid pGB853 contains the 80 kDa-encoding DNA under control of the lactase promoter.

Symbols as in fig. 15.

FIGURE 17: STRUCTURE OF pGB861

Plasmid pGB861 contains a factor VIII cDNA from which part of the B-domain has been removed.

▬▬▬▬▬▬ 92 kDa and 80 kDa-encoding DNA
ⅢⅢⅢⅢⅢⅢⅢⅢⅢⅢⅢ remaining part from B-domain

Other symbols as in fig. 15.

FIGURE 18: RNA ANALYSIS OF K. LACTIS TRANSFORMED WITH pGB850 AND pGB852

A: lane 1: Control K. lactis
        2: K. lactis transformed with pGB850
        3: Independent K. lactis transformed with pGB850

B. lane 1: Control K. lactis
        2: K. lactis transformed with pGB852
        3: Independent K. lactis transformed with pGB852

Arrows indicate the position of ribosomal RNA bands.

FIGURE 19: DETECTION OF 92 kDa AND 80 kDa SUBUNIT-CONTAINING PROTEINS PRODUCED BY TRANSFORMED K. LACTIS STRAINS WITH SPECIFIC MONO- AND POLYCLONAL ANTIBODIES

A. Immunoblot detection of the lactase-80 kDa fusion protein with polyclonal antibody RH 63275 and donkey anti-rabbit peroxidase. Lanes 1 and 3: cell extract of the parental K. lactis strain made in the presence of 0.05% Tween 80 or 2% NP40, respectively. Lanes 2 and 4: cell extract of K. lactis expressing the lactase-80 kDa fusion protein, made in the presence of 0.05% Tween 80 or 2% NP40, respectively. Lane 5 shows the pattern obtained with a factor VIII preparation. Arrow indicates the position of the fusion protein.

B. Immunoblot detection of the lactase-80 kDa fusion protein with polyclonal antibody RH 63272 and goat anti-rabbit alkaline phosphatase. Lanes 1 and 3: cell extract of the parental K. lactis strain made in the presence of 0.05% Tween 80 or 2% NP40, respectively. Lanes 2 and 4: cell extract of K. lactis transformed with pGB852 and expressing the lactase-80 kDa fusion protein, made in the presence of 0.05% Tween 80 or 2% NP40, respectively. Lane 5 shows the pattern obtained with a factor VIII preparation. Arrow indicates the position of the fusion protein.

C. Immunoblot detection of the lactase-80 kDa fusion protein with monoclonal antibody CLB-CAg 65 and goat anti-mouse alkaline phosphatase. All cell extracts were made in the presence of 0.05% Tween 80. Lanes 1-3: three independently isolated strains transformed with plasmid pGB850 containing the coding sequence for the 92 kDa subunit; lane 4 shows a factor VIII preparation; lanes 5-8 independently isolated strains transformed with plasmid pGB852 containing the coding sequence for the lactase-80 kDa fusion; lane 9: parental K. lactis strain. Arrow indicates the position of the fusion protein.

D. Immunoblot detection of the 92 kDa-prochymosin fusion with an antiserum against chymosin and donkey anti-rabbit peroxidase. Lane 1: parental K. lactis strain; lanes 2-4: independently isolated clones transformed with plasmid pGB851 containing the 92 kDa-prochymosin coding sequence; lane 5: yeast transformed with pGB850 containing the 92 kDa coding sequence only; lane 6: yeast transformed with pGB852 containing the 80 kDa coding sequence; lane 7: transformed yeast producing prochymosin; lane 8 shows the pattern of a commercial chymosin preparation. The cell extracts investigated in lanes 9-12 were incubated at pH 2 before preparing the samples. Lane 9: parental yeast strain; lanes 10-12: independently isolated clones transformed with the 92 kDa-prochymosin coding sequence. Arrow at left indicates position of prochymosin fusion. Arrows at right are at position of prochymosin and chymosin, respectively.

E. Immunoblot detection of the 80 kDA subunit protein with polyclonal antibody RH 63272. Lane 1: K. lactis transformed with pGB852 (cf Fig. 19B). Lanes 2-5: Independent K. lactis colonies transformed with pGB853. Lane 6: control strain.

F. Immunoblot detection of the 92 kDa sununit protein with monoclonal antibody CLB-CAg9. Lane 1: K. lactis transformed with pGB850. Lane 2: Control K. lactis.

G. Immunoblot detection of the 92 kDa and 80 kDa subunits expressed simultaneously in one cell. Lanes 1,2 and 3: detection of 80 kDa protein with polyclonal antibody RH 63272 Lane 1 control lane, lanes 2 and 3, two different transformed strains. Lanes 4,5 and 6 detection of 92 kDa protein with monoclonal antibody CLB-CAg9. Lane 4: control lane; lanes 5 and 6, same strains as in lanes 2 and 3.

H. Immunoblot detection of the 92 kDa-80 kDa fusion protein with polyclonal antibody RH 63272. Lane 1: Control K. lactis. Lanes 2-4: Independent K. lactis colonies transformed with pGB 861.

I. Immunoblot detection of secretion of the 92 kDa-80 kDa fusion protein with polyclonal antibody RH 63272. Lane 1: Plasma factor VIII. Lanes 2 and 3: cell supernatant and pellet of untransformed K. lactis. Lanes 4 and 5: cell supernatant and pellet of transformed K. lactis.

## DESCRIPTION OF THE SPECIFIC EMBODIMENTS

In accordance with the subject invention, novel DNA constructs are provided which include expression cassettes having an open reading frame encoding at least a portion of either or both the 80 kDa or 92 kDa subunits of factor VIII. The constructs will usually be prepared stepwise employing appropriate vectors, with cloning at each of the stages and analysis of the product to ensure that the proper organization and sequences have been obtained. The resulting constructs may then be introduced into appropriate vectors, particularly shuttle vectors, for transformation into preferred hosts and expression of the desired protein. The product may be expressed within the cytoplasm or may be secreted into the nutrient medium or a combination of both, where the unmatured protein which retains the signal leader may be further processed by appropriate enzymatic treatment. The resulting products are found to have biological and in many cases physiological activity, in being able to be used to promote clotting activity, either by themselves or in combination with endogenous factors in the patient. Alternatively, the subject products may be used to bind to the immunoglobulins which bind to factor VIII proteins in the patient, so as to prevent the immunoglobulins from diminishing the available factor VIII in the patient.

The hosts which are employed are microbial hosts, either prokaryotic or eukaryotic. Of particular interest for commercial fermentation are prokaryotic hosts of the Bacillus genus, more particularly B. subtilis or B. licheniformis. Among eukaryotes of particular interest are yeasts, particularly of the genus Saccharomyces and Kluyveromyces, more particularly Kluyveromyces, and preferably K. lactis. Therefore, in accordance with this invention, the constructs which are prepared and described will be exemplary for microbial organisms, but be directed to the preferred hosts, Bacillus and Kluyveromyces.

The particular hosts which are employed will desirably be industrial strains which are stable and have a high production. The hosts may be modified to enhance yields. For example, with the Bacillus strains, problems with exoproteases or plasmid instability may be rectified. The exoprotease problem may be corrected by employing exoprotease deficient mutants (Kawamura and Doi, 1984). While these authors modified B. subtilis, in the subject invention an improved strain of R. licheniformis has been produced (see Example 4). Where extrachromosomal maintenance is desired, opportunities for homologous recombination may be reduced by chromosomal deletion of sequences homologous to sequences present in the plasmid. There may also be an interest in removing promoters or other regulatory sequences from the chromosome common to the plasmid.

The proteins of interest are those which aid in correcting the clotting defect in providing a function lacking in the patient due to a defect in the endogenous factor VIII provision of the patient or due to endogenous antibodies which inactivate factor VIII (human), earlier defined as the combination of the 80 kDa and the 92 kDa proteins. The 80 kDa subunit extends to the carboxy terminus from Glu-1649 through Tyr-2332 and the 92 kDa subunit extends from Ala-1 through Arg-740, with a bridge between the two sequences having proteolytic cleavage sites. The bridge or central region referred to as the B-domain of factor VIII will range from Ser-741 through Arg-1648 (numbering according to Table 1).

The peptides of interest will include at least 10, more usually at least 12 amino acids. They may include the entire sequences of the 80 kDa and 92 kDa factor VIII subunits, and comprise fragments used to competitively bind antibodies to factor VIII, which will usually be homologous to a factor VIII sequence. Otherwise, usually less than 10%, more usually less than 5% of the amino acid sequence will be modified from the naturally occurring sequence by mutations, that is conservative or non-conservative substitutions, deletions or insertions. The smaller the fragment, the fewer the number of mutations which will be permissible, generally the total number of amino acids involved will be less than 15, more usually less than 10. A higher number of substitutions is permissible in cases where the substitutions are conservative, such as those amino acids which are aliphatic and which may be divided into groups which are neutral, polar, acidic or basic and those amino acids which are aromatic.

The proteins which are prepared may be divided into the following categories:

The first category consists of fragments from about 8 to 60, more usually from about 12 to 50 amino acids which are able to compete with factor VIII for antibodies, so as to serve to remove from the patient antibodies which may bind either to endogenous factor VIII or to factor VIII which is administered. These fragments may be used as fragments, or may be modified by joining them to various carriers, such as particles, other proteins, liposomes, or the like. The particular manner in which the fragments are modified will depend upon the mode of administration and the particular purpose for which the fragments are used.

The next group of proteins will be the subunits, which may be modified by truncation or extension, usually not exceeding 10% of the number of amino acids of the protein, where the proteins will be active in a complex of the two subunits to provide for clotting activity. In the case the subunits are biological subunits and may be administered individually, it is intended that the subunit forms a complex with an endogenous subunit giving active factor VIII. Alternatively, fused proteins may be prepared where all or a major portion ($>$90%) of a subunit may be fused to another protein, usually at least about 5 amino acids, particularly where the other protein may provide a signal sequence.

The next group of proteins will be proteins having sequences which individually are active in forming a clotting complex, but are connected, either head-to-head, head-to-tail, or tail-to-tail, either directly or by a bridge of fewer than about 900 amino acids, usually fewer than 500 amino acids, more usually fewer than about 60 amino acids. The bridge may be a synthetic bridge, where a portion of the natural bridge is removed, so as to substantially shorten the spacing between the two subunits as compared to the natural precursor. Also, the subunits may be truncated at their N- and/or C-termini, usually by not more than about 10 number %, more usually not more than about 5 number %. Desirably, the two subunits will be joined to each other or to the bridge by processing signals, which allow for enzymatic cleavage, particularly enzymatic cleavage in the intended patient, to provide for the individual fragments. Either the naturally occurring processing signal or an alternative processing signal may be present, there being a large number of protease signals recognized by human hosts, e.g., K-R, R-R, etc.

The coding sequence may be obtained in a variety of ways. The sequence for the factor VIII precursor has been

reported in the literature (see also table I). Following the procedures described in the literature, either a genomic sequence or a cDNA sequence might be prepared of all or a portion of the factor VIII subunits. Thus, exons, fragments or all of the cDNA for factor VIII precursor, synthetic sequences, or combinations thereof, may be employed for the encoding region. The sequences may be modified by in vitro mutagenesis, primer repair, resection, restriction, blunt ending, either by digestion or filling in, insertion of adaptors, insertion into polylinkers, ligation with linkers, and the like. Thus, numerous techniques have evolved for modifying a coding sequence, to provide for deletions, insertions, and substitutions, such as transitions and transversions. The preparation of the coding sequence may be done independently of the joining to the other members of the expression cassette or may be involved with the same process. Varying strategies may be employed, depending upon available sequences, vectors, the presence of restriction sites, or the like. Conveniently, the coding sequence may first be prepared, followed by joining to the other members of the expression cassette.

At the 5' terminus of the coding sequence, a signal leader may be introduced. The signal leader will serve to provide for secretion of the desired protein into the nutrient medium. The signal leader will normally be joined to the coding sequence by a processing signal, usually involving at least 2 codons, which code for a site which is recognized by an enzyme, which will cleave at that site. A large number of signal leaders have been studied in a variety of hosts. Signal leaders which have been employed in the preferred Bacillus strains are alpha-amylases, exoproteases and levansucrase. Signal sequences which have been employed in the yeast strains, particularly the Kluyveromyces strains, include alpha-factor, either Saccharomyces or Kluyveromyces alpha-factor, and synthetic leader sequences (see example 14).

The expression cassette will comprise a transcriptional and translational initiation region, joined to the 5' terminus of the coding sequence, so as to regulate transcription and translation of the coding sequence. Joined to the 3' end of the coding sequence will be a transcriptional and translational termination region, where the two regulatory regions will be functional in the intended expression host.

A number of transcriptional and translational initiation regions may be employed, but the regions will vary as to their efficiency in particular hosts and their effect on the growth characteristics of the particular host. Transcriptional and translational initiation region for Bacillus of particular interest are alpha-amylases, exoproteases and levansucrase. Transcriptional and translational initation regulatory regions for Kluyveromyces of particular interest are lactase, phosphoglycerate kinase, enolase etc. The termination regions are not as critical as the initiation regions, so that any convenient termination region may be employed. Thus, the termination region may be from the same gene as the transcriptional and translational initiation region or may be associated with a different gene, provided that the termination region is fuctional in the host. In many cases, the termination region may be associated with the gene from which the signal leader has been obtained or with a different gene.

The expression cassette may be used to transform the host directly or may be present as part of a vector. The vector is preferred since it provides for a number of conveniences. Most vectors will include one or more markers which allow for selection in the transformed host. Thus, one can select for those hosts which contain the expression construct as distinct from those hosts which have not been transformed. Markers for the most part provide for protection from biocides, particularly antibiotics, or impart prototrophy to an auxotrophic host. Illustrative markers include resistance to gentamycine (G418), kanamycin, tetracycline, ampicillin, chloramphenicol, neomycin etc. The resistance genes will have appropriate transcriptional and translational regulatory regions to function in the expression host.

The vector will also have a replication system which is functional in the host, which may be low or high copy number. Depending upon the nature of the construct, the vector may be safely maintained as an extrachromosomal element or may become integrated into the host chromosome, particularly where the vector has regions of homology between DNA sequences of the vector and DNA sequences of the host. Thus, integration can be encouraged, by providing for a region of homology between the vector and the host and particularly where the replication system which is chosen is unstable and can only be maintained under selective pressure.

Conveniently, the vector may be a shuttle vector, where after each manipulation, the vector may be transferred to a cloning host, such as E. coli. In this manner, the constructions may be amplified and analyzed to ensure that the desired results have been obtained.

Once the expression vector has been completed, it may then be used for transformation. Various techniques for transformation exist for the desired species. Both Bacillus and Kluyveromyces may be transformed employing protoplasts in the presence of a fusogen, particularly polyethylene glycol. Alternatively the plasmids may be linearized and K. lactis transformed by them in a medium containing a transformation agent e.g. lithium acetate and a biocide e.g. G418. Cells may then be regenerated and selected for the presence of the expression cassette. The transformants may then be grown in an appropriate culture medium for production of the desired products.

The desired products may be isolated in a variety of ways. Where the product is retained in the cytoplasm, the cells may be harvested, lysed, and the protein extracted using liquid-liquid extraction, chromatography, electrophoresis, or other conventional techniques. Where the product is secreted, the nutrient medium may be continuously circulated or partially withdrawn, and the desired product obtained as described with the lysed cells.

Once the product has been obtained into the desired purity, it may be brought in a pharmaceutical preparation adapted to its intended purpose. For use with a patient, it may be combined with a variety of physiologically acceptable additives, such as water, saline, phosphate buffered saline, or the like and administered to the patient, usually by injection, particularly intravascularly. The amount administered will vary widely depending upon the purpose of the protein, the status of the patient, the protein involved, its activity, as well as other conventional considerations. For the most part, the physiologically active subunits will be administered such that a level is attained of from about 0.1 to about 10 units per ml of blood.

Another aspect of the invention is the preparation of antibodies that recognize factor VIII subunit proteins. They may be prepared, using conventional techniques, by immunizing mammals with the proteins subject of the invention. Antibodies are obtained specific to the 80 kDa proteins and other to the 92 kDa proteins. When recovered from the blood they can be used in the production of hybridomas from which monoclonal antibodies can be produced. The antibodies of the present invention can be used in the diagnosis of factor VIII abnormalities, using any of the conventional competitive or direct assays on blood samples, which comprises bringing the sample into contact with the produced antibody and assaying for the presence or absence of a conjugate of the antibody with factor VIII antigen as a measure of the factor VIII protein in the sample.

The antibodies can also be used for the purification of factor VIII proteins which comprises bringing a liquid phase comprising protein and a content of impurities into contact with a solid phase on which the produced antibody is immobilized, separating the liquid phase from the solid phase and eluting from the solid phase protein with a reduced content of impurities.

The following examples are offered by way of illustration and not by way of limitation.

EXAMPLES

General Methods

1. Cloning techniques

For general cloning techniques reference may be made to the handbook of Maniatis et al., 1982.

Restriction enzymes are used as recommended by the manufacturerer and are obtained either from New England Biolabs (Biolabs), Bethesda Research Laboratories (BRL) or Boehringer Mannheim (Boehringer). In general 1 to 5 units of enzyme are needed to cleave 1 microgram of DNA.

For ligation, DNA fragments are run on an agarose gel, electroeluted, purified over a NACS column (BRL) as recommended by the manufacturer, ethanol precipitated and incubated in a buffer containing 50mM Tris-HCl pH7.4; 10 mM $MgCl_2$; 10mM dithiothreitol; 1mM ATP and T4 DNA ligase (Biolabs).

For end-labeling, DNA cut with a restriction enzyme is phenol-extracted and ethanol-precipitated. The phosphate is removed from the 5' end with Calf Intestine Alkaline Phosphatase (Boehringer) in 50mM Tris-HCl pH8.0 at 37°C. After inactivation of the enzyme at 65°C for 1 hr, followed by phenol extraction and ethanol precipitation, the DNA is labeled using gamma-$^{32}$P-ATP (1-2 micromolar) in a buffer containing 50mM Tris-HCl, pH 7.6; 10mM $MgCl_2$; 5mM

dithiothreitol for 30 minutes at 37°C. Unincorporated nucleotides are removed by Sephadex G-50 chromatography.

Filling in of 5' overhanging restriction sites was done with the large fragment of DNA Polymerase I (Biolabs). Incubation was for 15 minutes at 30°C in a buffer containing 10mM Tris-HCl pH7.7; 50mM NaCl; 10mM MgCl$_2$; 1mM dithiothreitol and unlabeled nucleotides at 20 micromolar.

Transformation of E. coli was either by the classical CaCl$_2$ technique (Maniatis et al., 1982) or exactly as described by Hanahan (1983). Transformation of Bacillus or Kluyveromyces with foreign DNA was as described in the text.

2. Protein analysis. Sample preparation and electrophoresis

To 40 microliters of Kluyveromyces cell extract 10 microliters of sample buffer (0.313 M Tris-HCl pH6.8, 10% Sodium dodecylsulphate (SDS), 50% glycerol), 2.5 microliters of a saturated solution of Bromophenol Blue (BPB) in water and 2.5 microliters of beta-mercaptoethanol were added. The samples were boiled for 2 min, centrifuged in an Eppendorf centrifuge for 2 min and subsequently layered on polyacrylamide gels according to Laemli (1970), consisting of a 7.5% separation gel and a 5% stacking gel. The gels were run at 60 mA until the BPB marker reached the bottom of the gel.

Samples of Bacillus cell extracts and extracellular proteins were dissolved in sample buffer (0.06M Tris-HCl pH6.8; 2% SDS; 10% glycerol). BPB and beta-mercaptoethanol were added as above. Samples were boiled for 5 min, centrifuged and subsequently layered and run on a 6% polyacrylamide gel (5% stacking gel).

All gels depicted were run from top to bottom.

3. Protein blotting procedure

The proteins from the gel were blotted (Western blot) onto nitrocellulose filters (0.45 micrometers) using a Biorad electroblot apparatus and a buffer containing 21 mM Tris; 160 mM glycine and 20% methanol, during 16 hrs at 150 mA. The blots were treated during 1 hr with a Bovine Serum Albumin (BSA)-solution (1% BSA in 50 mM Tris-HCl, pH7.5; 0.9% NaCl) and subsequently for 2.5 hrs with an antiserum in fresh BSA-solution. After 3 times rinsing with Tween 20 buffer (50 mM Tris-HCl, pH7.5; 0.9% NaCl and 0.05% Tween 20) the second antibody in fresh BSA-solution was added and incubated for 2 hrs followed again by 3 rinses with Tween 20 buffer. The blots were stained depending on the enzyme coupled to the second antibody. In the case of horse-radish peroxidase staining was for 20 minutes with 0.6 mg/ml 4-chloro-naphthol and 0.015% $H_2O_2$ in a buffer containing 50 mM Tris-HCl, pH7.5 and 0.9% NaCl followed by rinsing in distilled water. When alkaline phosphatase was used the blot was stained during a 15 min incubation with 0.033% nitro blue tetrazolium and 0.017% 5-bromo-4-chloro-3-indolyl-phosphate in 100 mM Tris-HCl, pH9.5; 100 mM NaCl; 5 mM $MgCl_2$.

## 4. Determination of chymosin

In the case of the 92 kDa-prochymosin fusion product part of the samples were treated at low pH in order to activate chymosin. To 500 microliters of cell extract 4M HCl was added to lower the pH to approximately 2.0 and the sample was incubated at room temperature for 2 hrs. The pH was adjusted to 6.4 with 2.5M Tris. The samples were used for analyses on polyacrylamide gels and for determination of chymosin activity according to a standard milk clotting assay (Foltman, 1970).

## 5. Preparation and characterization of monoclonal and polyclonal antibodies to factor VIII

Balb/c mice were immunized with purified human factor VIII-von Willebrand factor complex. Purification is performed by an agarose gel filtration of cryoprecipitate or a factor VIII concentrate used for therapeutical purposes (Central Laboratory of The Netherlands Red Cross Blood Transfusion Service, Amsterdam, The Netherlands) as described by Van Mourik and Mochtar (1970). Lymphocyte hybridization was performed as described by Galfré et al. (1977). Description of the techniques used for selection of clones producing monoclonal antibodies to factor VIII have been provided elsewhere (Stel et al., 1983).

Monoclonal antibodies to factor VIII were identified according to their reactivity with factor VIII polypeptides using a procedure adapted from Towbin et al. (1979). Batches as described above were first analyzed by SDS-polyacrylamide gel electrophoresis (Laemli, 1970). Then, separated proteins were transferred electrophoretically (Western transfer) from the gel into a nitrocellulose sheet. The sheets were washed and incubated for 2 hrs with monoclonal antibodies and washed again. Then, the sheets were incubated with peroxidase-conjugated goat-antimouse immunoglobulins, washed and incubated with 4-chloro-1-naphthol and $H_2O_2$ to detect antibody-bound peroxidase (see also above).

This procedure demonstrated that the following distinct monoclonal antibodies had been raised (Fig. 1). The antibody, designated CLB-CAg9, reacted with a polypeptide of 92 kDa and larger polypeptides. One antibody, CLB-CAg 65 reacted with the doublet of 79-80 kDa. The latter antibody did not react with the 92 kDa polypeptide and larger polypeptides. This demonstrates that the 79-80 kDa doublet possesses an epitope which is not present on the 92 kDa polypeptide.

In addition, monoclonal antibodies to factor VIII were identified according to their reactivity with factor VIII polypeptides, purified by immunoaffinity chromatography as previously described (Stel, 1984). The major portion of this preparation, devoid of von Willebrand factor, consists of the 79-80 kDa doublet. Traces of larger polypeptides are also present. Analysis of this preparation by immunoblotting demonstrated that, besides monoclonal antibody CLB-CAg 9, also the antibodies CLB-CAg 21 and CLB-CAg 62 reacted with the 92 kDa and larger polypeptides.

To obtain polyclonal antibodies to factor VIII polypeptides, rabbits were immunized by standard procedures with the factor VIII preparation, purified by chromatography as described above. The antibodies thus obtained were identified by immunoblotting as described above using purified factor VIII-von Willebrand factor or purified factor VIII polypeptides, separated by SDS-polyacrylamide gel electrophoresis and subsequently transferred to nitrocellulose sheets as target proteins. This analysis demonstrated that three distinct polyclonal antisera had been raised (RH 63271, RH 63272 and RH 63275). These antisera reacted with the 79-80 kDa doublet, the 92 kDa polypeptide and larger polypeptides. It also shows that these antisera react with smaller polypeptides.


Example 1


SYNTHESIS OF A cDNA TO FACTOR VIII mRNA


Isolation of human liver polyA+ RNA:


During work with RNA care was taken to work Ribonuclease free. Sterile, disposable plasticware was used and all glassware was rendered nuclease free by heating overnight at 180°C. Wherever possible, solutions were incubated with 0.1% diethylpyrocarbonate for 30 min and autoclaved.

Total RNA from 60 g of human liver tissue was prepared by the guanidium thiocyanate method as described by Chargwin et al. (1979). Homogenization of finely grounded frozen liver tissue in guanidium thiocyanate was done with a Polytron for 45 sec. The yield of total RNA was approximately 133 mg. The isolation of polyA+ RNA was done by a modification of the procedure outlined by Aviv and Leder (1972). Total RNA was bound "batchwise" to an appropriate amount of oligo(dT)-cellulose (Collaborative Research, type T2) in 50 mM Tris-HCl (pH 7.5); 1 mM EDTA; 0.2 % SDS; 0.4 M NaCl. A column was prepared from this preparation and polyA+ RNA was eluted with the same buffer lacking NaCl. A second cycle of absorption of polyA+ RNA to oligo(dT)-cellulose was performed using less stringent conditions (buffer containing 0.25 M NaCl), in order to separate polyA+ RNA from remaining ribosomal RNA. The final yield of polyA+ RNA was about 830 micrograms. Analysis of this preparation by agarose gel electrophoresis showed that it did not contain ribosomal RNA.

Primer-directed cDNA synthesis:

Synthesis of factor VIII cDNA was based on the method of Gubler and Hoffman (1983) as modified by Toole et al. (1984). Specific primers for cDNA synthesis were synthesized with a Biosearch DNA synthesizer (Figure 2). Human liver polyA$^+$ RNA (20 micrograms in distilled water) was mixed with a 10-fold molar excess of single stranded primer in 10mM CH$_3$HgOH in a total volume of 35 microliters and incubated for 10 min at room temperature. Beta-mercaptoethanol was added to a final concentration of 47 mM and then RNasin (Promega-Biotec) to 100 U/ml (total volume 47 microliters). Then 50 microliters of 2xRT buffer (0.1M Tris-HCl pH8.3 (43°C); 10mM MgCl$_2$; 150mM KCl; 60mM beta-mercaptoethanol; 1mM each of dATP, dGTP and dTTP and 0.2mM of dCTP) and 0.4 microliters of water were added. After 2 min incubation at 50°C followed by 2 min at 43°C, 2.6 microliters of super RT (Anglian Biotechnology, 17 U/microliter) were added. First strand synthesis was for 45 min at 43°C. The reaction was stopped on ice and 300 microliters of a solution containing 40mM Tris-HCl (pH7.5); 10mM MgCl$_2$; 75mM KCl and 25 micrograms/ml BSA (BRL, Desoxyribonuclease and Ribonuclease free) were added. For second strand labeling 2 microliters of alpha-$^{32}$P-dCTP (3000Ci/mmol; 10mCi/ml) were added, followed by 8 microliters of DNA Polymerase I (10 U/microliter; Biolabs) and 4 microliters of RNase H (10 U/microliter; Anglian). After 60 min at 11°C, 4 microliters of 20mM ATP and 1 microliter of T4 DNA ligase (400 U/microliter; Biolabs) were added and incubation continued for 120 min at 18°C. The reaction was stopped by the addition of 30 microliters of 0.5M EDTA and 4 microliters of 10% SDS. The mixture was extracted with phenol and the DNA precipitated with isopropanol. Unincorporated nucleotides were removed on a Sephadex G-50 column. The final yield was 400 ng of double stranded cDNA.

Isolation of partial factor VIII cDNA clones:

The double stranded cDNA was digested with a combination of two restriction enzymes as indicated in Figure 2. After digestion, cDNA fragments were separated according to size on a Sepharose CL-4B column. Samples of fractions from the column were run on an agarose gel and the length estimated by autoradiography. Material larger than 600 bp was used for subsequent cloning experiments. Fragments A and B were ligated into pAT153 (Twigg and Sherrat, 1980) C and D in pACYC177 (Chang and Cohen, 1978) and E in pUC19 (Yanish-Perron et al., 1985). Before ligation to cDNA fragments, all plasmids were digested with the corresponding two restriction enzymes and the vector fragment isolated by agarose gel electrophoresis, followed by electroelution and DEAE-Sephacel chromatography.

After ligation, E. coli DH1 was transformed exactly as described by Hanahan (1983). About 100 colonies with plasmids containing an insert were obtained per nanogram of cDNA. Colonies were transferred to nitrocellulose filters (Grunstein and Hogness, 1975), lysed and hybridized to radioactively labeled probes (Figure 2). Clones which were derived from cDNA synthesis with fragment 1 as primer were analysed with fragment 2. Clones synthesized with fragment 2 were analysed with fragment 3, etc. Labeling of fragments was with gamma-32P-ATP and polynucleotide kinase (Maniatis et al., 1982). Prehybridization of filters was at 20°C in a solution containing 6 x SSC, 5 x Denhardt's (0.1% Ficoll; 0.1% polyvinyl pyrrolidone; 0.1% BSA) and 100 micrograms of E. coli DNA per ml. Hybridization was in the same solution containing 10 ng of labeled fragment per ml overnight at 20°C. Filters were washed in 6 x SSC at 37°C and autoradiographed. About 1 in 1000 colonies was positive and correct clones were isolated for all cDNA subfragments. Clones from fragments A, B, C, D and E (Figure 2) were called pCLB81, pCLB82, pCLB83, pCLB84 and pCLB85, respectively.

Construction of a full length factor VIII cDNA:

Clone pCLB85 is missing a small DNA fragment from the factor VIII-encoding sequence from the ATG start codon up to the SacI site at position 16 (Table I). The missing nucleotides were provided by chemical synthesis of a DNA fragment thereby creating a SalI site upstream of the ATG codon such that it reads 5'GTCGACATGCAAATAGAGCTC3'. To this end pBR322 was cleaved with AvaI and the AvaI site was filled in with the large fragment of DNA Polymerase I. After a second cleavage with PvuII the remaining pBR322 fragment was circularized giving plasmid PI. The 1.9kb factor VIII cDNA-specific BamHI-SacI fragment from pCLB85 was ligated to PI cleaved with BamHI and BanII. After ligation a chemically synthesized DNA fragment (CGGTCGACATGCAAATAGAGCT) was added, and the mixture was incubated with the large fragment of DNA polymerase I and all four desoxyribonucleotides. After phenol extraction, followed by digestion with SalI and another phenol extraction the fragment was ligated. The resulting plasmid pCLB86 now contains a SalI site in front of the factor VIII ATG start codon.

Plasmids pCLB81, 82, 83, 84 and 86 are now assembled to a full length factor VIII clone in 3 steps. First, the inserts of pCLB84 and 86 are combined in pUC19 cleaved with SalI and PstI (pCLB87). Then, plasmids pCLB81 and 82 are cleaved with EcoRI plus HpaI and BamHI plus EcoRI, respectively, and ligated into pEP121 (Enger-Valk, 1984) giving pCLB88. Then, the inserts of pCLB83, 87 and 88 are combined to a cDNA clone containing the complete protein-encoding part of the factor VIII gene as a SalI-HpaI fragment in pEP121 (pCLB89).

The factor VIII cDNA insert in pCLB89 was sequenced according to the methods of Sanger et al. (1977) and Maxam and Gilbert (1980). The sequence is shown in Table I.


Example 2


CONSTRUCTION OF A BACILLUS SECRETION VECTOR WITH THE BACILLUS LICHENIFORMIS ALPHA AMYLASE GENE EXPRESSION CASSETTE


Cloning of the alpha-amylase gene from B. licheniformis strain T5 in the pUB110 (Matsumura et al., 1984) vector yielded the recombinant plasmids pGB33 and pGB34 as described in European patent application EP-A-0134048.

The inserted B. licheniformis restriction fragments from pGB33 to be sequenced were subcloned into the multicloning site of M13mp8 and M13mp9 (Messing and Vieira, 1982) and nucleotide sequences were determined by the di-deoxy chain termination method of Sanger et al (1977).

The promoter, Shine-Dalgarno sequence and the signal sequence of the cloned alpha-amylase gene are described in European patent application EP-A-0224294.

In order to place a gene for bovine chymosin behind this alpha-amylase transcriptional and translational initiation regulating sequences we digested pGB34 and pUR1523, an E. coli plasmid that harbours the gene for bovine chymosin (see European patent application EP-A-0077109), with PstI. After ligation and transformation to competent B. subtilis 1A-

40 (BGSC, Ohio U.S.A.) cells, plasmid DNA was isolated from selected transformants. After restriction endonuclease analyses the correct recombinant plasmid called pLC28 was obtained. Cutting pLC28 with the restriction endonuclease ClaI, followed by resection with the exonuclease Bal-31, ligation and transformation yielded pLC83 (see European patent application EP-A-0134048).

From recent literature (Ulmanen et al. (1985), Shiroza et al. (1985)) it was known that the level of secretion and production of a heterologous gene fused to an alpha-amylase based secretion vector is also depending on the codon position of the mature alpha-amylase at which the heterologous gene is joined. Therefore, we incubated a M13mp8 alpha-amylase harbouring clone (called pPB4) with SstII followed by resection with the exonuclease Bal-31, cutting with SmaI, ligation and transformation to competent E. coli JM101 cells (Yanisch-Perron et al., 1985). This procedure yielded a so-called alpha-amylase "leaderbank". This means a set of M13mp8 derived clones where the number of amino acids (called n) behind the maturation site of the alpha-amylase protein varies.

The M13mp8 "leader" clones with the correct reading frame have been used to place the (pro)chymosin gene in phase behind the alpha-amylase transcriptional, translational and secretion regulatory sequences. These fusion plasmids were called pLCn for the chymosin constructs and pLPn for the prochymosin constructs, respectively, where n stands for the number of alpha-amylase amino acids behind the maturation site.

The pLCn and pLPn constructs were modified in such a way, that the EcoRI site, orginating from plasmid pGB33 was removed by exonuclease Bal-31 action after cutting with EcoRI.

Furthermore, the pLC constructs have been modified in such a way that they contain unique restriction endonuclease sites directly behind the "leader" sequences, and a recognition site for signal peptidase directly behind the "leader" sequences. To fulfill these two requirements four oligodeoxyribonucleotides, I to IV, were chemically synthesized:

a. I   :   5'-AATTCGCGGCCGCC-3'        (no. 104 1526 GBR 22774)
   II  :   5'-CGGGTCGACTCAAGCTTA-3'    (no. 104 1527 GBR 22775)
   III :   3'-GCGCCGGCGGGCCCAGCT-5'    (no. 104 1524 GBR 22773)
   IV  :   3'-GAGTTCGAATTTAA-5         (no. 104 1523 GBR 22772)

b.    EcoRI      XmaIII    XmaI/SmaI  SalI      HindIII


5' AA    TTC GCG  GCC GCC   CGG   GTC   GAC   TCA   AGC  TTA  3'


$\longleftarrow$————— I—————$\longrightarrow$$\longleftarrow$————— II—————$\longrightarrow$


3' G CGC   CGG CGG   GCC   CAG   CTG   AGT   TCG  AAT  TTA A 5'


$\longleftarrow$————— III—————$\longrightarrow$$\longleftarrow$————— IV—$\longrightarrow$


      glu phe ala ala ala arg val asp ser ser leu asn ser


These fragments constitute the multicloning site and they were joined to pLC constructs yielding the secretion vectors of the pOL5-n type (Fig. 3). This plasmid serves as a vehicle for subsequent introduction (see Example 3) of expressible genes encoding proteins with factor VIII activity, the plasmid being suitable for transforming Bacillus. n in the plasmid designation refers to the number of amino acids from the mature alpha amylase present behind the signal sequence, Fig. 3 illustrating a plasmid that will produce 21 amino acids in this section. The number of amino acids in this section does not affect the ability of the plasmid, when carrying a factor VIII protein gene to express factor VIII protein in a Bacillus transformant. Bacillus subtilis was transformed with pOL5-21 and the transformant deposited on 14 July 1986 in the Centraal Bureau voor Schimmelcultures (the CBS), at Oosterstraat 1, 3742 SK  BAARN, NETHERLANDS where it has been given the deposit number CBS-304.86.

By using the pOL5-n expression vector having sequences homologous with the host chromosome of B. licheniformis T5 strain we observed both segregational and structural instability of the plasmids.

To circumvent homologous recombination between the alpha-amylase sequences on the B. licheniformis host cell chromosome on one hand and the same sequences on the expression vector we have decided to delete these sequences.

Firstly we constructed a Bacillus licheniformis host strain which lacks the alpha-amylase promoter and the complete alpha-amylase chromosomal gene (see Example 4).

Secondly we removed from the expression vector the remaining homologous sequences upstream the alpha-amylase promoter. Therefore the pOL5-n vectors were digested by EcoRV and BglI, ligated and transformed to protoplasts of Bacillus subtilis 1A40 (BGSC, Ohio U.S.A.). Plasmid DNA was isolated from neomycin resistant transformants. After restriction endonuclease analyses the right recombinant plasmids designated pOL5-delta were obtained (see Figure 4).

Protoplasts of Bacillus licheniformis T9 strain (see Example 4) transformed with these pOL5-delta vectors yielded neomycin resistant transformants. These T9 transformants were subjected to a plasmid stability test as described by Andreoli, 1985.

The plasmid DNA population was analysed in more detail by restriction enzyme mapping. After about 50 generations less than 5% of the pOL5-delta vectors have been cured in contrast to T9 cells harbouring pOL5 plasmids where up to 90% of the plasmids have been cured.

These results indicate that the pOL5-delta expression vectors are stable in the genetic background of the T9 Bacillus licheniformis host.

Example 3


INTRODUCTION OF THE HUMAN FACTOR VIII cDNA INTO A BACILLUS
VECTOR AND ANALYSIS OF EXPRESSION OF THE 92 kDa FACTOR VIII
SUBUNIT IN BACILLUS SUBTILIS.


In pOL5-21 the 92 kDa subunit gene is inserted
yielding pOL92 (see Figure 5A,B). After transformation of
protoplasts from Bacillus subtilis (Chang and Cohen, 1979)
followed by colony hybridization analysis (Grunstein and
Hogness, 1975) and plasmid restriction enzyme mapping, we
obtained the constructs pOL92-T-21 and pOL92-A-21,
respectively.

To examine the expression of factor VIII
polypeptides, Bacillus subtilis transformants were grown in an
appropriate medium e.g. Trypton soya broth at 37-40°C with
aeration in the presence of neomycin (10 micrograms/ml).
Samples of culture were centrifuged and lysed as follows:
After centrifugation of a 2 ml culture, cells were resuspended
in 0.1 ml of a buffer containing 50mM Tris-HCl, pH7.5 and 50mM
EDTA. One microliter of 100mM PMSF was added and egg white
lysozyme to a final concentration of 2mg/ml. After 5 min at
37°C another microliter of 100mM PMSF was added and incubation
continued for 5 min at 37°C. The reaction was terminated by
the addition of sample buffer.

The SDS-polyacrylamide gel analysis of these samples
is shown in Fig. 6. When analyzed by SDS-polyacrylamide gel
the cell lysate from pOL92-T-21 contained bands of molecular
weights of 46 kDa, 58 kDa and 88 kDa, respectively, which are
much stronger than in the control.  The molecular weight of
the largest pOL92-T-21 encoded protein is 88 kDa. Based on the
amino acid composition the large subunit polypeptide has a
molecular weight of 85 kDa (Vehar et al., 1985), while for the
human fragmentation product a molecular weight of 92 kDa was
measured.  Differences in molecular weight might be accounted for partly by experimental error, by differences in protein modifying reactions like glycosylation, and/or differences in processing of the translated protein products. Thus, this result demonstrates that we have produced large subunit factor VIII protein in <u>Bacillus</u>.

Bacillus <u>subtilis</u> transformed with pOL92-T-21 was deposited in the CBS on 14 July 1986 where it was given the deposit number CBS-303.86.

<p align="center">Example 4</p>

<p align="center">CONSTRUCTION OF AN EXOPROTEASE DEFICIENT AND ALPHA-AMYLASE<br>DEFICIENT <u>BACILLUS LICHENIFORMIS</u> HOST</p>

Bacillus <u>licheniformis</u> produces at least two major extracellular proteases, an alkaline protease (or subtilisin) and a neutral protease. Therefore strain T5 (European patent application EP-A-0134048) was mutagenized by two subsequent cycles with ultraviolet radiation according to the method of Miller (1972) and plated on Spizizen glucose minimal medium supplemented with 0.4% casein. Seven of 12.000 colonies screened produced a smaller halo after the first cycle. These seven mutants were subjected to the second cycle of UV mutagenesis. Two of 10.000 colonies screened showed no halo or extracellular protease activity on the 0.4% casein plates. These double mutants were characterized for both total exoprotease production in liquid cultures as described by Kawamura and Doi (1984) as well as the alpha-amylase production by measuring the production of reducing sugars from starch (Ghandi and Kjaergaard, 1975) to exclude the isolation of mutants defective in secretion. One such isolate, which was alpha-amylase proficient and of which the exoprotease activity was beyond detection was designated strain T6.

The next step was to construct a <u>Bacillus</u> <u>licheniformis</u> T6 derivative which lacks the alpha-amylase promoter and the complete alpha-amylase structural gene. To create such deletion mutation (designated delta Amy) the recombinant plasmid pGB33 as described in European patent application EP-A-0134048 was digested with Hind III and BglI, ligated and transformed to competent cells from Bacillus subtilis strain 1A40 (BGSC, Ohio, U.S.A.). Plasmid DNA was isolated from neomycin resistant alpha-amylase negative transformants. After restriction endonuclease analyses the correct recombinant delta Amy plasmid called pGB33delta was obtained. This plasmid contains portions of the 5' and 3' flanking sequences of the Bacillus licheniformis alpha-amylase gene. To replace the wild-type alpha-amylase gene with the delta Amy deletion mutation the pGB33delta plasmid was transformed into Bacillus licheniformis T6 protoplasts as described in Example 5. Two of 20.000 neomycin resistant transformants showed no extracellular alpha-amylase activity and retained the pGB33delta plasmid, indicating that the frequency of gene conversion was very low. The two alpha-amylase deficient $Nm^R$ transformants were cured of the plasmid by growing for 20 generations in minimal medium as described by Meyer and Fiechter (1985) in the absence of antibiotic selection.

Neomycin sensitive cells cured of the plasmid appeared at a frequency of about 2%. One of these colonies was designated strain T9. We used Southern blotting and hybridization (Maniatis et al. 1982) to confirm that the T9 strain carried the 1.9 kb deletion of the alpha-amylase gene. The construction of the alpha-amylase deficient Bacillus licheniformis T9 host is presented in Figure 7.

An additional advantage of this T9 strain is when this host strain harbours a Bacillus licheniformis alpha-amylase secretion vector e.g. pOL5 (see Example 2) there exists no longer competition for binding RNA polymerase molecules between the chromosomal alpha-amylase promoter and the alpha-amylase promoter located on the plasmid DNA.

Example 5


PROTOPLAST TRANSFORMATION AND REGENERATION OF BACILLUS
LICHENIFORMIS STRAINS.

Bacillus licheniformis T5 is an industrial strain
used for the production of extracellular enzymes, which has
been described in European Patent application EP-A-0134048.

Bacillus licheniformis strain T5 was grown overnight
in 50 ml NBSG-X medium (Thorne and Stull, 1966) at 37°C. The
culture was diluted 1:1 with fresh NBSG-X medium and grown for
another 1-1.5 hrs. After centrifuging for 10 min at 5000 rpm
in a Sorvall type GSA rotor and resuspending in 10 ml of SMM
buffer, containing 1.5M sucrose; 0.06M $MgCl_2$ and 0.06M
maleate, the cells were protoplasted by incubating for 2 hrs
at 37°C in the presence of 2 mg/ml of lysozyme.
The protoplasts were spun down (10 min at 5000 rpm),
resuspended in 5 ml of SMML buffer (L-broth in which 1.5M
sucrose; 0.06M $MgCl_2$ and 0.06M maleate has been dissolved),
mixed and repelleted. After being resuspended, the protoplasts
were incubated for 2 min in 30% polyethylene glycol 6000 with
1 microgram of plasmid pGB33 (see Example 2).

After this incubation a 1:3 dilution with SMML
medium was carried out and, after centrifugation, the
protoplast-containing pellet was resuspended in 1 ml of SMML
medium. Then, 0.1 ml aliquots were plated on regeneration agar
plates containing 0.7% (w/v) $K_2HPO_4$; 0.3% (w/v) $KH_2PO_4$; 0.125%
(w/v) $(NH_4)_2SO_4$; 0.035% (w/v) $MgSO_4.7H_2O$; 1.5% (w/v) agar;
3.7% (w/v) KCl; 0.1% (w/v) glucose; 0.01% (w/v) BSA
supplemented with 0.1% (w/v) spore solution co taining 0.2%
(w/v) $MnSO_4$;  0.2% (w/v) $ZnSO_4$; 0.2% (w/v) $CoCl_2$; 0.5% (w/v)
$FeSO_4$; 6% (w/v) NaCl and 0.5% (w/v) $CaCl_2$. Moreover, these
plates contained 10 micrograms/ml of neomycin.

After incubation at 37°C for at least 72 hrs, the
plates were replica plated on heart-infusion agar plates containing 10 micrograms/ml of neomycin. Transformants were analysed on their DNA content by isolation of DNA as described by Holmes and Quigley (1981) followed by characterisation on agarose gels. Among the transformants 5% were found not to contain any plasmid DNA, but they contained the complete pGB33 DNA as an integral part of their genome. Plasmid pGB33 was found to have been integrated into the chromosome by homologous recombination between the alpha-amylase sequences on the B. licheniformis T5 chromosome on one hand and the same sequences on the plasmid pGB33 on the other hand.


Example 6


CONSTRUCTION OF A BACILLUS INDUSTRIAL STRAIN SUITED FOR EFFICIENT CHROMOSOMAL INTEGRATION OF HETEROLOGOUS GENES.


The plasmid pBC16 is derived from Bacillus cereus and is highly homologous to pUB110 as described by Polak and Novick (1982). The main difference between pBC16 and pUB110 is that pBC16 contains a tetracyclin resistance gene where pUB110 has a neomycin resistance gene.

B. licheniformis T5 transformants, as described in Example 5, harbouring two amylase genes and one copy of pUB110 in their chromosome, were transformed using pBC16 linearized with XbaI, according to the protoplast transformation protocol as described in Example 5. 10 micrograms/ml of tetracyclin were included in the regeneration plates. Selection for tetracyclin resistant colonies resulted in a strain where the chromosomally located neomycin gene derived from pUB110 was replaced by the tetracyclin gene of pBC16 by double reciprocal recombination.

The resulting strain T5-16 can be used as a suitable strain for efficient chromosomal insertion of pUB110-derived plasmid constructs. Strain T5-16 is tetracyclin resistant and neomycin sensitive, and has approximately 3kb of DNA sequences homologous to pUB110 in its genome.

To obtain an exoprotease deficient B. licheniformis strain suited for chromosomal integration of heterologous genes the strain T5-16 and strain T6 (see Example 4) were protoplasted and fused as described in European patent application EP-A-0134048.

The obtained tetracyclin resistant fusants were screened for exoprotease activities and characterized by genomic analysis using $^{32}$P-labeled alpha-amylase encoding DNA probe. A fusant, which is tetracyclin resistant, exoprotease negative and has approximately 3kb of DNA sequences homologous to pUB110 in its genome, is designated strain T6-16.

Example 7

INTEGRATION OF THE HUMAN FACTOR VIII GENE INTO THE BACILLUS LICHENIFORMIS CHROMOSOME; EXPRESSION AND SECRETION OF 92 kDa FACTOR VIII SUBUNIT.

Bacillus licheniformis strain T5-16 was transformed with the plasmid pOL92-T-21 and pOL92-A-21 according to the protocol as described in Example 5. Transformants were selected on regeneration plates containing 5 micrograms/ml of neomycin. None of the transformants was found to contain plasmid DNA, in all cases integration of plasmid DNA by homologous recombination into the genome of strain T5-16 occurred.

To examine the expression of factor VIII polypeptides these T5-16 transformants were grown in heart-infusion medium, supplemented with 0.5% of starch. After 3 days of incubation at 40°C samples for analysis on protein gels were prepared as follows: 3 ml of fermentation broth was centrifuged and from the supernatant fraction protein was precipitated by addition of 5% trichloroacetic acid (TCA); the precipitated material was resuspended in 1% TCA and pelleted again by centrifugation. The protein from 25 ml of supernatant was then dissolved in 0.5 ml of sample buffer. The pellet fraction of 6 ml of culture broth was dissolved in 0.7 ml of sample buffer. The composition of both supernatant and cell pellet fraction was studied by analysing 60 and 50 microliters, respectively, on 6% SDS-polyacrylamide gel as shown in Figure 8.

From the result it can be concluded that Bacillus licheniformis can synthesize and secrete factor VIII 92 kDa proteins as intact products because a protein band is clearly visible in cell pellet and supernatant protein fractions of B. licheniformis T5-16 cells transformed with pOL92-A-21 and pOL92-T-21, while in the control (strain T5-16) these bands are not seen.

Example 8

SYNTHESIS AND SECRETION OF THE 80 kDa AND 92 kDa SUBUNIT PROTEINS USING THE BACILLUS LICHENIFORMIS ALPHA-AMYLASE SECRETION VECTOR pOL5-DELTA

To create a Bacillus expression plasmid containing the 80 kDa subunit protein the following three DNA fragments were combined:

(i) the 4.6 kb SalI-BalI fragment from pOL5-40 delta containing the alpha-amylase promoter, leader, terminator, the neomycin resistance gene and a Bacillus origin of replication.

(ii) a 0.6 kb SalI-HincII fragment from a M13mp18-80 kDa clone containing the N terminal part of the 80 kDa subunit preceded by an ATG codon and a SalI restriction site as described in Example 13.

(iii) a 2.3 kb HincII-EcoRV fragment from pGB852 containing most of the 80 kDa coding sequence and the lactase terminator.

After ligation of these three fragments the recombined DNA molecules are introduced in Bacillus by means of protoplast transformation.

Plasmid DNA was isolated from neomycin resistant transformants as described by Andreoli (1985) followed by restriction endonuclease mapping. The plasmid searched for was called pOL80-T-40delta and its structure is shown in Figure 9.

To examine the expression of factor VIII 80 kDa protein the obtained Bacillus subtilis 1A40 transformants were grown in Trypton soya broth medium with aeration in the presence of neomycin (10 micrograms/ml).

After overnight cultivation at 37°C protein samples were prepared and analysed as described in the General Methods section. Figure 10C shows the detection of the 80 kDa protein in the cell lysates of pOL80delta B. subtilis transformants in contrast to the pOL5delta control strains. This result demonstrates that we have produced factor VIII 80 kDa protein in Bacillus subtilis.

To remove the homologous DNA sequences between the Bacillus licheniformis chromosome of the T9 strain and the plasmid pOL92-T-21 we followed the same procedure as described in Example 2 for the construction of the expression vector pOL5delta. The plasmid thus obtained, pOL92-T-21delta, has a structure as shown in Figure 5C.

Also the 7.4 kb SalI-HpaI fragment from pCLB89 containing the full length factor VIII cDNA and the 5.8 kb SalI-EcoRV fragment from pGB861 containing a fusion between the 92 kDa and 80 kDa proteins (see Example 14) were introduced in the alpha-amylase secretion vector pOL5delta in a similar way as described in Example 3. The plasmids thus obtained were called pOLF8-2delta and pOL92/80-21delta, respectively.

To examine the expression of factor VIII polypeptides the Bacillus T9 transformants harbouring pOL5-21delta, pOL80-T-40delta and pOL92-T-21delta, respectively, were grown in double strength minimal medium (Anagnostopoulos and Spizizen, 1961) supplemented with 0.5% of yeast extract. After two days of cultivation at 40°C protein samples were prepared for analysis on SDS-polyacrylamide gel, blotted onto a nitrocellulose filter and incubated with appropriate antibodies as described in the General Methods section. From the results shown in Figure 10A and 10B it can be concluded that transformed Bacillus licheniformis T9 can synthesize and secrete factor VIII specific 92 kDa and 80 kDa polypeptides, respectively.


Example 9


EXPRESSING A FUSION PROTEIN BETWEEN THE 92 kDa AND 80 kDa FACTOR VIII SUBUNITS USING A BACILLUS PLASMID CONSTRUCT


After fermentation of Bacillus transformants harbouring respectively pOLF8-2delta and pOL92/80-21delta (see Example 8) only traces of factor VIII specific polypeptides could be detected.

The factor VIII DNA encoding a 92/80 kDa fusion protein was put behind the strong Bacillus licheniformis 3-4 promoter (see EP-A-0224294) as follows:

The 5.6 kb SalI-NsiI fragment encoding the 92/80 kDa fusion protein from pGB861, the 3.9 kb XbaI-XmaIII fragment encoding the 3-4 promoter, the neomycin resistance gene and an oligonucleotide with the sequence 5'dGGCCTGCA were ligated and transformed to protoplasts of Bacillus. This transformation yielded the plasmid pPROM92/80PB (see Figure 11).

Bacillus strains harbouring pPROM92/80PB were cultured for at least 2 days at 40°C as described in Example 8 and protein samples were prepared and analysed for expression of factor VIII polypeptides as described in the General Methods section.

Figure 10D shows the detection of the 92/80 kDa fusion protein in the cell lysate of pPROM92/80PB Bacillus transformants in contrast to the Bacillus control strains.

Example 10

CONSTRUCTION OF PLASMID pGBdLAC CONTAINING THE LACTASE
PROMOTER AND TERMINATOR SEQUENCES.

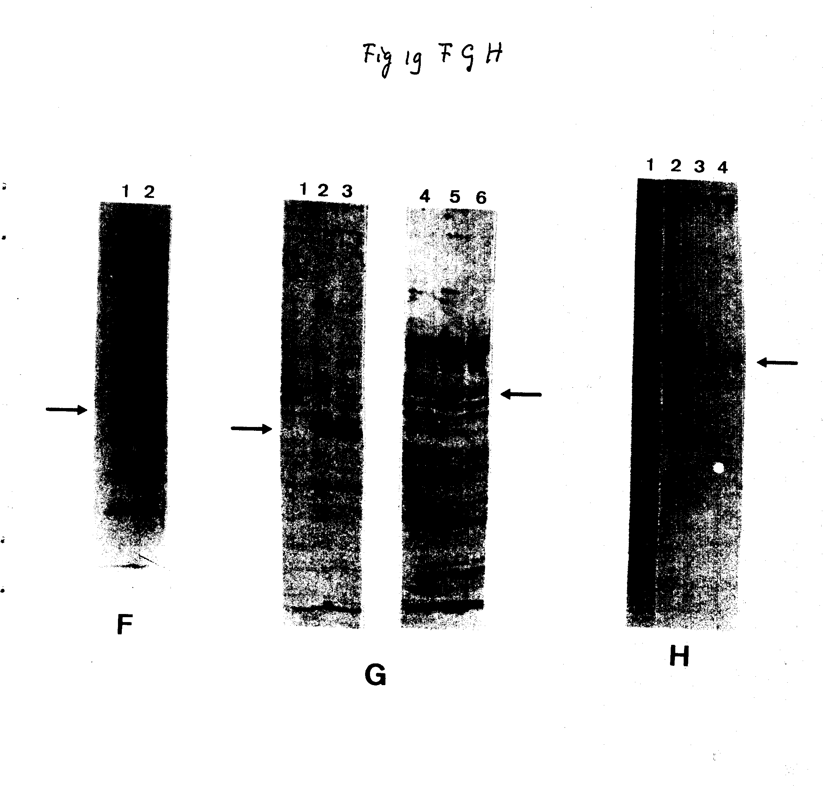

Chromosomal DNA was isolated from Kluyveromyces lactis strain CBS 2360 (Das and Hollenberg, 1982), cleaved with XhoI, and separated according to size on a sucrose gradient. Fractions containing the lactase gene were detected with a LAC4 probe (Breunig et al., 1984) after spotting the DNA on a nitrocellulose filter. The lactase gene containing DNA was cloned into the XhoI site of plasmid pPA153-215 (Andreoli, 1985) giving rise to plasmid pPA31. An XbaI fragment of pPA31 containing the lactase gene was subcloned in the XbaI site of pUC19 (Yanisch-Perron et al., 1985) which yields plasmid pUCla56. The EcoRI-XbaI fragment containing the 3' end of the lactase gene (Breunig et al., 1984) was cloned as a separate fragment in pUC19. In the EcoRI-XbaI fragment a ClaI site is present which is normally methylated in a dam + strain and is about 100 nucleotides downstream of the EcoRI site. To be able to cut at this ClaI site, the plasmid was transfected to a dam(-) E. coli strain, e.g. strain GM113 (Arraj and Marinus, 1983; Marinus and Morris, 1973). Plasmid DNA was obtained and the ClaI-XbaI fragment containing the 3' end of the gene isolated. In addition, the XbaI-ClaI fragment containing the 5' end of the lactase gene was isolated from pUCla56. Both fragments were simultaneously ligated into the XbaI site of pUC19, giving rise to plasmid pGBdLAC (Fig. 12).

Example 11

CONSTRUCTION OF A K. LACTIS EXPRESSION VECTOR CONTAINING THE
92 kDa FACTOR VIII SUBUNIT.

As a first step in the synthesis of a 92 kDa subunit expression plasmid the 1.4kb Hind III-PstI fragment, containing the 3' end of the lactase gene (Breunig et al., 1984), was ligated onto the 0.4kb BamHI-Hind III fragment (nucleotides 1875 to 2283 as in Table I) of factor VIII cDNA and ligated into plasmid pSP65 (Melton et al., 1984) cleaved with BamHI and PstI, giving rise to plasmid pGB840. Next, a stopcodon which is in phase with the factor VIII reading frame was introduced into the Hind III site. To this end an oligonucleotide with the sequence 5' AGCTGAGGGCCCTC was synthesized which contains in addition to the TGA stopcodon an ApaI recognition site (GGGCCC). It was ligated into the Hind III site of pGB840 yielding plasmid pGB841. From the latter plasmid the 1.2kb BamHI-EcoRV fragment was isolated and ligated into the 7.4kb BamHI-EcoRV fragment of plasmid pCLB86. This gives rise to plasmid pGB842 containing the complete 92 kDa subunit encoding DNA containing a stopcodon at the Hind III site in pAT153. To create a K. lactis expression plasmid containing the 92 kDa subunit protein the following 3 fragments were combined:

(i) the 3kb SalI-EcoRV fragment from pGB842 containing the 92 kDa-subunit DNA and the lactase terminator.

(ii) A 0.25kb SnaBI-SalI fragment containing the part of the lactase promoter from position - 282 (Breunig et al., 1984) to position -26 at which a SalI linker was introduced by the method described before (EP-A-0096430).

(iii) A 8.3kb SnaBI-EcoRV fragment from a derivative of pGBdLAC containing a G418 resistance gene (see EP-A-0096430). The resulting plasmid was called pGB850 (Fig. 13).

E. coli transformed with pGB850 was deposited in the CBS on 14 July 1986 where it was given the deposit number CBS-305.86.

Example 12

CONSTRUCTION OF A K. LACTIS EXPRESSION VECTOR CONTAINING A FUSION BETWEEN THE 92 kDa-SUBUNIT AND PROCHYMOSIN.

The prochymosin cDNA was put in the same reading frame behind the BamHI site at position 1875 of the factor VIII cDNA (Table I) as follows: The 11.0 kb EcoRV-BamHI

fragment of pGB850 was isolated and the BamHI site filled in using the Klenow fragment of DNA polymerase I and all four deoxynucleotide triphosphates. Next, the 2.3kb SalI-HindIII fragment containing the prochymosin gene from plasmid pGB131 (EP-A-0096430) was isolated and the SalI and Hind III sites filled in as above. Both fragments were ligated yielding plasmid pGB851 which contains the prochymosin-encoding DNA downstream of the 92 kDa-encoding DNA (Fig. 14).

Example 13

CONSTRUCTION OF K. LACTIS EXPRESSION VECTORS FOR THE 80 kDa SUBUNIT PROTEIN.

A vector was constructed for the expression of a lactase-80 kDa fusion protein. Two fragments were used for the 80 kDa expression plasmid:
(i) A derivative of plasmid pGBdLAC containing the G418 resistance gene (above) was linearized with ClaI.
(ii) Factor VIII plasmid pCLB88 was cleaved with BamHI and HpaI and the 2.7kb fragment from position 4749 to position 7439 in the factor VIII cDNA sequence (Table I) was isolated. Both fragments were made blunt using the Klenow fragment of DNA polymerase I and all four deoxyribonucleotides, ligated and E. coli HB101 transformed. Plasmids containing the 80 kDa-encoding part in the right orientation and in frame behind the lactase-encoding sequences were identified by restriction enzyme digestions and DNA sequence analysis according to Maxam and Gilbert (1980). The resulting plasmid pGB852 is shown in Fig. 15.

E. coli transformed with pGB852 was deposited in the CBS on 14 July 1986 where it was given the deposit number CBS-306.86.

In another expression vector an ATG codon preceded by a SalI restriction site was introduced in front of the protease cleavage site of the 80 kDa subunit protein:

a BamHI-HindII fragment from position 4749 to position 5584 of
the factor VIII cDNA sequence was introduced in M13mp18
(Messing and Vieira, 1982) cleaved with BamHI and HindII.
Single stranded phage DNA was isolated and hybridized to a
chemically synthesized nucleotide with the sequence
3' TTTGCGGTAGTTGCCAGCTGTACCTTTATTGAGCATGA 5'.
The phage DNA was rendered partially double-stranded by
simultaneous hybridization to plasmid DNA of M13mp18 cleaved
with BamHI and HindII.

After hybridization gaps were filled in by incubation with the
Klenow fragment of DNA polymerase I (Boehringer) in a buffer
containing all four deoxyribonucleotides. E. coli JM101 was
transformed with the resulting plasmid DNA and correct
colonies were picked up by hybridization to the
oligonucleotide which was end-labelled by gamma-(32)P-ATP and
T4 polynucleotide kinase. Plasmid DNA was isolated and the
mutagenized 0.6kb SalI-HindII fragment was isolated. It was
ligated to the 2.3kb HindII-EcoRV fragment containing most of
the 80 kDa coding sequence from pGB852 and the 9.2kb SalI-
EcoRV fragment containing the pUC sequence from pGB850. The
resulting 80 kDa expression plasmid pGB853 was isolated
(figure 16).


Example 14


CONSTRUCTION OF K. LACTIS VECTORS EXPRESSING A FUSION BETWEEN
THE 92 kDa AND 80 kDa PROTEINS


In the fusion vector the PstI site at position 2660 of the
factor VIII sequence was ligated to the BamHI site at position
4749 as follows:
The 2.6kb SalI-PstI fragment encoding the 92 kDa protein from
pCLB88, the 3.6kb BamHI-HindII fragment from pGB852, pUC19
digested with SalI and HindIII and an oligonucleotide with the
sequence ACGTCTAG were ligated giving rise to plasmid pGB860.
A derivative of pGB850 was made in which the SalI site in the polylinker was removed by a partial digestion with SalI followed by a 1 minute incubation with Bal31 as recommended by the manufacturer (BRL) yielding pGB850 dHSX. Next the factor VIII DNA encoding the fusion protein was put behind the lactase promoter by ligating the 5.8kb SalI-EcoRV fragment from pGB860 to the 92kb SalI-EcoRV fragment from pGB850 dHSX, resulting in the expression vector pGB861 (figure 17).

A vector for secretion of factor VIII subunits was obtained by replacing the leader for secretion of factor VIII in pGB 861 by a synthetic leader sequence as follows: the 1.9kb SalI-BamHI fragment encoding part of the 92 kDa protein of pGB861 was recloned in pTZ18R (United States Biochemical Corporation). A MluI site was introduced in the DNA at position 2 and 3 of the factor VIII protein sequence by using an oligonucleotide of the sequence

5' ACCCAGGTAGTATCTacgcgtGGCACTAAAGCAGAA 3'.

The oligonucleotide was hybridized to the single stranded phage DNA of the plasmid containing the SalI-BamHI fragment in pTZ18R. After hybridization the gaps were filled in, and plasmids containing the oligonucleotide isolated as described in Example 10. From one of the correct plasmids the mutagenized 1.7kb SalI-BglII fragment was used to replace the corresponding fragment from pGB861, giving rise to pGB862. Plasmid pGB863 was cleaved with SalI and MluI. A synthetic DNA fragment was made encoding the synthetic leader sequence:

5' TCGACATGGCTTTCAGATCCTTGTTGGCTTTGTCCGGTTT...
   3' GTACCGAAAGTCTAGGAACAACCGAAACAGGCCAAA...

...GTCCTGTGGTGCTTTGGCTGCTA3'
...CAGGACACCACGAAACCGACGATCGCG5'

It was ligated into pGB863 cleaved with SalI and BamHI giving rise to plasmid pGB864.

## Example 15

TRANSFORMATION OF <u>KLUYVEROMYCES</u> <u>LACTIS</u> AND ANALYSIS OF THE RNA AND PROTEIN PRODUCTS OF THE TRANSFORMANTS.

All expression plasmids were linearized at the unique SacII restriction site and <u>K. lactis</u> was transformed according to Ito et al. (1984) using 0.2M lithium acetate, and 25 micrograms/ml of G418. Transformants were analysed by isolation of chromosomal DNA followed by digestion with various restriction enzymes. Cell lines containing intact copies of the plasmid DNA integrated in the chromosome were grown up for 3 days in YEPD medium at 30°C for RNA and protein analysis.

For RNA analysis cells were harvested for 10 min at 5000 rpm. The pellet was suspended to an $OD_{610nm}$ of 300 in a buffer containing 100 mM Tris-HCl, pH7.5; 100 mM LiCl; 0.1 mM EDTA and 0.5 mg/ml of heparin. To 1 ml of cells 2 g of glass beads (diameter 0.25-0.30 mm) were added and the suspension was vortexed 5 times 30 seconds. Glass beads and cell debris were removed by centrifugation at 10,000 rpm for 5 min. The pellet was re-extracted with 0.5 ml of the above buffer and centrifuged. The supernatants were pooled, phenol extracted and ethanol precipitated. The RNA was incubated with glyoxal, (McMaster and Carmichal, 1977), run on an agarose gel and blotted to Gene Screen Plus (NEN Research Products) as described by NEN. The result is shown in Figure 16. It is clear that both in <u>K. lactis</u> transformed with pGB850 or pGB852, RNA of the expected size can be found.

For protein analysis cells were harvested by a low speed centrifugation and suspended in 0.9% NaCl; 1mM phenylmethylsulfonyl fluoride (PMSF) (and 0.05% Tween 80 or 2% Nonidet P40 when indicated) to a final optical density of 500 at 610 nm. In a 10 ml conical test tube 1.2 g of glass beads (diameter 0.25-0.30 mm) were added to 0.6 ml of the yeast cell suspension and the suspension was vortexed for 1 minute. The cell debris and glass beads were removed by a low speed centrifugation. Samples were prepared and run on a SDS-polyacrylamide gel, blotted onto a nitrocellulose filter and incubated with antibodies as described in the General Methods section.

Figure 19A shows the detection of the lactase-80 kDa fusion protein produced by K. lactis cells transformed with pGB852 as shown with the polyclonal antiserum RH 63275 and donkey anti-rabbit peroxidase (Promega Biotec). A band corresponding to a protein product of the expected size (about 100 kDa) can be seen in lane 2 and, although much weaker, also in lane 4. In the parental K. lactis strain (lanes 1 and 3) this band cannot be detected.

The same results were obtained in a parallel experiment where a blot with the same samples was incubated with polyclonal antiserum RH 63272 and goat anti-rabbit alkaline phosphatase from Promega Biotec (Figure 19B).

In a third experiment the presence of the fusion product could be shown by using monoclonal antiserum CLB-CAg 65 and goat anti-mouse alkaline phosphatase (Figure 19C). In lanes 6-9 four independent K. lactis clones transformed with plasmid pGB852 are analysed each producing the lactase-80 kDa fusion product. They clearly possess a protein product of the expected size (approximately 100 kDa) not seen in the parental yeast strain (lane 9) nor in K. lactis transformed with plasmid pGB850 containing the DNA sequence for the 92 kDa subunit of factor VIII (lanes 1-4).

For the detection of the 92 kDa-prochymosin fusion commercially available polyclonal antibodies (Chr. Hansen's laboratory) against chymosin were used (Figure 19D). Lanes 2-4 show the pattern of three independently obtained transformants of K. lactis with pGB851. A protein product of approximately the expected size (110 kDa) can be seen. After treatment of the cell extracts at pH 2 (General Methods, 4) still most of the immuno-reacting material is located at the same position (lanes 10-12) but also two minor bands at the approximate position of chymosin are visualised. So the fusion product is only partially processed by the pH 2 treatment. In the milk clotting assay chymosin activity (0.1 mg/l) is found in contrast to the parental yeast strain and the other controls, demonstrating the presence of activated chymosin.

In another series of experiments it was shown that K. lactis also expresses 80 kDa and 92 kDa subunit proteins separately. In Fig. 19E a cell lysate of K. lactis transformed with pGB 853, run on a SDS polyacrylamide gel and blotted to a nitrocellulose filter is shown. The blot was incubated with RH 63272 and goat anti-rabbit alkaline phosphatase. In Fig. 19F a similar experiment in which K. lactis was transformed with pGB 850 and monoclonal CLB-CAg 9 was used after blotting, is shown. Moreover, the two subunit proteins can be expressed simultaneously in one cell (Fig. 19G). For this experiment pGB 850 and pGB 853 were cut with Sac II, and, after phenolization, mixed in equimolar amounts, and ligated. K. lactis was transformed with a mixture in which more than 50% of the DNA was in dimers. After growing up, cells were lysed and strains expressing both subunits selected by immunoblotting and incubation with RH 63272 and CLB-CAg-9.

Fig. 19H shows that K. lactis is also able to express fusions between 92 kDa and 80 kDa proteins. Cells were transformed with pGB 861, lysed, proteins run on a SDS polyacrylamide gel and analyzed with RH 63272. In most transformants a fusion protein of the expected size was found.

Secretion of a fusion protein between 92 kDa and 80 kDa subunits is demonstrated in Fig. 19I. K. lactis was transformed with pGB864 and intra- and extracellular proteins determined separately.

It can be concluded that K. lactis can synthesize as intact products both the 80 kDA and 92 kDa proteins separately as well as fused to one another both intra- and extracellularly.

In accordance with the subject invention, a number of factor VIII related proteins may be prepared by transformed microorganisms, including the 80 kDa and 92 kDa subunit proteins, a fusion protein of the 80 kDa and 92 kDa subunit proteins joined by a truncated bridge and fusion proteins of the subunit with an endogenous protein, where the endogenous protein is the N-terminus. The above products may be cytoplasmic or secreted in the medium. Individual products may be produced in the same or different cells and in the same or different cellular hosts.

It is evident from the above results, that the subject invention provides for products and methods which produce biologically and physiologically active proteins having factor VIII activity or being able to compete with factor VIII for antibodies which inhibit such activity. Thus, methods are provided using microbial hosts, with their inherent economies and effiencies for the production of a complex protein, so as to provide drugs which can be used in place of the natural drug, so as to provide a stable, uniform, safe supply of factor VIII related products.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

## Table I

The factor VIII cDNA insert of pCLB89. The amino acid sequence
Ala-1 through Tyr-2332 of factor VIII and its signal sequence
Met(-19) through Ser(-1) with the corresponding sequence of
nucleotides. Phe-973 is encoded by the codon TTC.

```
             10        20        30        40        50        60
     GTCGACATGCAAATAGAGCTCTCCACCTGCTTCTTTCTGTGCCTTTTGCGATTCTGCTTT
     -19 M  Q  I  E  L  S  T  C  F  F  L  C  L  L  R  F  C  F  -2

             70        80        90       100       110       120
     AGTGCCACCAGAAGATACTACCTGGGTGCAGTGGAACTGTCATGGGACTATATGCAAAGT
       S  A  T  R  R  Y  Y  L  G  A  V  E  L  S  W  D  Y  M  Q  S  19

            130       140       150       160       170       180
     GATCTCGGTGAGCTGCCTGTGGACGCAAGATTTCCTCCTAGAGTGCCAAAATCTTTTCCA
       D  L  G  E  L  P  V  D  A  R  F  P  P  R  V  P  K  S  F  P

            190       200       210       220       230       240
     TTCAACACCTCAGTCGTGTACAAAAAGACTCTGTTTGTAGAATTCACGGATCACCTTTTC
       F  N  T  S  V  V  Y  K  K  T  L  F  V  E  F  T  D  H  L  F  59

            250       260       270       280       290       300
     AACATCGCTAAGCCAAGGCCACCCTGGATGGGTCTGCTAGGTCCTACCATCCAGGCTGAG
       N  I  A  K  P  R  P  P  W  M  G  L  L  G  P  T  I  Q  A  E

            310       320       330       340       350       360
     GTTTATGATACAGTGGTCATTACACTTAAGAACATGGCTTCCCATCCTGTCAGTCTTCAT
       V  Y  D  T  V  V  I  T  L  K  N  M  A  S  H  P  V  S  L  H  99

            370       380       390       400       410       420
     GCTGTTGGTGTATCCTACTGGAAAGCTTCTGAGGGAGCTGAATATGATGATCAGACCAGT
       A  V  G  V  S  Y  W  K  A  S  E  G  A  E  Y  D  D  Q  T  S

            430       440       450       460       470       480
     CAAAGGGAGAAAGAAGATGATAAAGTCTTCCCTGGTGGAAGCCATACATATGTCTGGCAG
       Q  R  E  K  E  D  D  K  V  F  P  G  G  S  H  T  Y  V  W  Q  139

            490       500       510       520       530       540
     GTCCTGAAAGAGAATGGTCCAATGGCCTCTGACCCACTGTGCCTTACCTACTCATATCTT
       V  L  K  E  N  G  P  M  A  S  D  P  L  C  L  T  Y  S  Y  L

            550       560       570       580       590       600
     TCTCATGTGGACCTGGTAAAAGACTTGAATTCAGGCCTCATTGGAGCCCTACTAGTATGT
       S  H  V  D  L  V  K  D  L  N  S  G  L  I  G  A  L  L  V  C  179

            610       620       630       640       650       660
     AGAGAAGGGAGTCTGGCCAAGGAAAAGACACAGACCTTGCACAAATTTATACTACTTTTT
       R  E  G  S  L  A  K  E  K  T  Q  T  L  H  K  F  I  L  L  F

            670       680       690       700       710       720
     GCTGTATTTGATGAAGGGAAAAGTTGGCACTCAGAAACAAAGAACTCCTTGATGCAGGAT
       A  V  F  D  E  G  K  S  W  H  S  E  T  K  N  S  L  M  Q  D  219

            730       740       750       760       770       780
     AGGGATGCTGCATCTGCTCGGGCCTGGCCTAAAATGCACACAGTCAATGGTTATGTAAAC
       R  D  A  A  S  A  R  A  W  P  K  M  H  T  V  N  G  Y  V  N

            790       800       810       820       830       840
     AGGTCTCTGCCAGGTCTGATTGGATGCCACAGGAAATCAGTCTATTGGCATGTGATTGGA
       R  S  L  P  G  L  I  G  C  H  R  K  S  V  Y  W  H  V  I  G  259

            850       860       870       880       890       900
     ATGGGCACCACTCCTGAAGTGCACTCAATATTCCTCGAAGGTCACACATTTCTTGTGAGG
       M  G  T  T  P  E  V  H  S  I  F  L  E  G  H  T  F  L  V  R
```

```
         910       920       930       940       950       960
AACCATCGCCAGGCGTCCTTGGAAATCTCGCCAATAACTTTCCTTACTGCTCAAACACTC
 N  H  R  Q  A  S  L  E  I  S  P  I  T  F  L  T  A  Q  T  L   299

         970       980       990      1000      1010      1020
TTGATGGACCTTGGACAGTTTCTACTGTTTTGTCATATCTCTTCCCACCAACATGATGGC
 L  M  D  L  G  Q  F  L  L  F  C  H  I  S  S  H  Q  H  D  G

        1030      1040      1050      1060      1070      1080
ATGGAAGCTTATGTCAAAGTAGACAGCTGTCCAGAGGAACCCCAACTACGAATGAAAAAT
 M  E  A  Y  V  K  V  D  S  C  P  E  E  P  Q  L  R  M  K  N   339

        1090      1100      1110      1120      1130      1140
AATGAAGAAGCGGAAGACTATGATGATGATCTTACTGATTCTGAAATGGATGTGGTCAGG
 N  E  E  A  E  D  Y  D  D  D  L  T  D  S  E  M  D  V  V  R

        1150      1160      1170      1180      1190      1200
TTTGATGATGACAACTCTCCTTCCTTTATCCAAATTCGCTCAGTTGCCAAGAAGCATCCT
 F  D  D  D  N  S  P  S  F  I  Q  I  R  S  V  A  K  K  H  P   379

        1210      1220      1230      1240      1250      1260
AAAACTTGGGTACATTACATTGCTGCTGAAGAGGAGGACTGGGACTATGCTCCCTTAGTC
 K  T  W  V  H  Y  I  A  A  E  E  E  D  W  D  Y  A  P  L  V

        1270      1280      1290      1300      1310      1320
CTCGCCCCCGATGACAGAAGTTATAAAAGTCAATATTTGAACAATGGCCCTCAGCGGATT
 L  A  P  D  D  R  S  Y  K  S  Q  Y  L  N  N  G  P  Q  R  I   419

        1330      1340      1350      1360      1370      1380
GGTAGGAAGTACAAAAAAGTCCGATTTATGGCATACACAGATGAAACCTTTAAGACTCGT
 G  R  K  Y  K  K  V  R  F  M  A  Y  T  D  E  T  F  K  T  R

        1390      1400      1410      1420      1430      1440
GAAGCTATTCAGCATGAATCAGGAATCTTGGGACCTTTACTTTATGGGGAAGTTGGAGAC
 E  A  I  Q  H  E  S  G  I  L  G  P  L  L  Y  G  E  V  G  D   459

        1450      1460      1470      1480      1490      1500
ACACTGTTGATTATATTTAAGAATCAAGCAAGCAGACCATATAACATCTACCCTCACGGA
 T  L  L  I  I  F  K  N  Q  A  S  R  P  Y  N  I  Y  P  H  G

        1510      1520      1530      1540      1550      1560
ATCACTGATGTCCGTCCTTTGTATTCAAGGAGATTACCAAAAGGTGTAAAACATTTGAAG
 I  T  D  V  R  P  L  Y  S  R  R  L  P  K  G  V  K  H  L  K   499

        1570      1580      1590      1600      1610      1620
GATTTTCCAATTCTGCCAGGAGAAATATTCAAATATAAATGGACAGTGACTGTAGAAGAT
 D  F  P  I  L  P  G  E  I  F  K  Y  K  W  T  V  T  V  E  D

        1630      1640      1650      1660      1670      1680
GGGCCAACTAAATCAGATCCTCGGTGCCTGACCCGCTATTACTCTAGTTTCGTTAATATG
 G  P  T  K  S  D  P  R  C  L  T  R  Y  Y  S  S  F  V  N  M   539

        1690      1700      1710      1720      1730      1740
GAGAGAGATCTAGCTTCAGGACTCATTGGCCCTCTCCTCATCTGCTACAAAGAATCTGTA
 E  R  D  L  A  S  G  L  I  G  P  L  L  I  C  Y  K  E  S  V

        1750      1760      1770      1780      1790      1800
GATCAAAGAGGAAACCAGATAATGTCAGACAAGAGGAATGTCATCCTGTTTTCTGTATTT
 D  Q  R  G  N  Q  I  M  S  D  K  R  N  V  I  L  F  S  V  F   579
```

```
          1810      1820      1830      1840      1850      1860
GATGAGAACCGAAGCTGGTACCTCACAGAGAATATACAACGCTTTCTCCCCAATCCAGCT
 D   E   N   R   S   W   Y   L   T   E   N   I   Q   R   F   L   P   N   P   A

          1870      1880      1890      1900      1910      1920
GGAGTGCAGCTTGAGGATCCAGAGTTCCAAGCCTCCAACATCATGCACAGCATCAATGGC          619
 G   V   Q   L   E   D   P   E   F   Q   A   S   N   I   M   H   S   I   N   G

          1930      1940      1950      1960      1970      1980
TATGTTTTTGATAGTTTGCAGTTGTCAGTTTGTTTGCATGAGGTGGCATACTGGTACATT
 Y   V   F   D   S   L   Q   L   S   V   C   L   H   E   V   A   Y   W   Y   I

          1990      2000      2010      2020      2030      2040
CTAAGCATTGGAGCACAGACTGACTTCCTTTCTGTCTTCTTCTGGATATACCTTCAAA          659
 L   S   I   G   A   Q   T   D   F   L   S   V   F   F   S   G   Y   T   F   K

          2050      2060      2070      2080      2090      2100
CACAAAATGGTCTATGAAGACACACTCACCCTATTCCCATTCTCAGGAGAAACTGTCTTC
 H   K   M   V   Y   E   D   T   L   T   L   F   P   F   S   G   E   T   V   F

          2110      2120      2130      2140      2150      2160
ATGTCGATGGAAAACCCAGGTCTATGGATTCTGGGGGTGCCACAACTCAGACTTTCGGAAC          699
 M   S   M   E   N   P   G   L   W   I   L   G   C   H   N   S   D   F   R   N

          2170      2180      2190      2200      2210      2220
AGAGGCATGACCGCCTTACTGAAGGTTTCTAGTTGTGACAAGAACACTGGTGATTATTAC
 R   G   M   T   A   L   L   K   V   S   S   C   D   K   N   T   G   D   Y   Y
                                               12

          2230      2240      2250      2260      2270      2280
GAGGACAGTTATGAAGATATTTCAGCATACTTGCTGAGTAAAAACAATGCCATTGAACCA          739
 E   D   S   Y   E   D   I   S   A   Y   L   L   S   K   N   N   A   I   E   P

          2290      2300      2310      2320      2330      2340
AGAAGCTTCTCCCAGAATTCAAGACACCCTAGCACTAGGCAAAAGCAATTTAATGCCACC
 R   S   F   S   Q   N   S   R   H   P   S   T   R   Q   K   Q   F   N   A   T

          2350      2360      2370      2380      2390      2400
ACAATTCCAGAAAATGACATAGAGAAGACTGACCCTTGGTTTGCACACAGAACACCTATG          779
 T   I   P   E   N   D   I   E   K   T   D   P   W   F   A   H   R   T   P   M

          2410      2420      2430      2440      2450      2460
CCTAAAATACAAAATGTCTCCTCTAGTGATTTGTTGATGCTCTTGCGACAGAGTCCTACT
 P   K   I   Q   N   V   S   S   S   D   L   L   M   L   L   R   Q   S   P   T

          2470      2480      2490      2500      2510      2520
CCACATGGGCTATCCTTATCTGATCTCCAAGAAGCCAAATATGAGACTTTTTCTGATGAT          819
 P   H   G   L   S   L   S   D   L   Q   E   A   K   Y   E   T   F   S   D   D

          2530      2540      2550      2560      2570      2580
CCATCACCTGGAGCAATAGACAGTAATAACAGCCTGTCTGAAATGACACACTTCAGGCCA
 P   S   P   G   A   I   D   S   N   N   S   L   S   E   M   T   H   F   R   P

          2590      2600      2610      2620      2630      2640
CAGCTCCATCACAGTGGGGACATGGTATTTACCCCTGAGTCAGGCCTCCAATTAAGATTA          859
 Q   L   H   H   S   G   D   M   V   F   T   P   E   S   G   L   Q   L   R   L

          2650      2660      2670      2680      2690      2700
AATGAGAAACTGGGGACAACTGCAGCAACAGAGTTGAAGAAACTTGATTTCAAAGTTTCT
 N   E   K   L   G   T   T   A   A   T   E   L   K   K   L   D   F   K   V   S
```

```
        2710      2720      2730      2740      2750      2760
AGTACATCAAATAATCTGATTTCAACAATTCCATCAGACAATTTGGCAGCAGGTACTGAT
 S   T   S   N   N   L   I   S   T   I   P   S   D   N   L   A   A   G   T   D      899

        2770      2780      2790      2800      2810      2820
AATACAAGTTCCTTAGGACCCCCAAGTATGCCAGTTCATTATGATAGTCAATTAGATACC
 N   T   S   S   L   G   P   P   S   M   P   V   H   Y   D   S   Q   L   D   T

        2830      2840      2850      2860      2870      2880
ACTCTATTTGGCAAAAAGTCATCTCCCCTTACTGAGTCTGGTGGACCTCTGAGCTTGAGT
 T   L   F   G   K   K   S   S   P   L   T   E   S   G   G   P   L   S   L   S      939

        2890      2900      2910      2920      2930      2940
GAAGAAAATAATGATTCAAAGTTGTTAGAATCAGGTTTAATGAATAGCCAAGAAAGTTCA
 E   E   N   N   D   S   K   L   L   E   S   G   L   M   N   S   Q   E   S   S

        2950      2960      2970      2980      2990      3000
TGGGGAAAAAATGTATCGTCAACAGAGAGTGGTAGGTTATTCAAAGGGAAAAGAGCTCAT
 W   G   K   N   V   S   S   T   E   S   G   R   L   F   K   G   K   R   A   H      979

        3010      3020      3030      3040      3050      3060
GGACCTGCTTTGTTGACTAAAGATAATGCCTTATTCAAAGTTAGCATCTCTTTGTTAAAG
 G   P   A   L   L   T   K   D   N   A   L   F   K   V   S   I   S   L   L   K

        3070      3080      3090      3100      3110      3120
ACAAACAAAACTTCCAATAATTCAGCAACTAATAGAAAGACTCACATTGATGGCCCATCA
 T   N   K   T   S   N   N   S   A   T   N   R   K   T   H   I   D   G   P   S      1019

        3130      3140      3150      3160      3170      3180
TTATTAATTGAGAATAGTCCATCAGTCTGGCAAAATATATTAGAAAGTGACACTGAGTTT
 L   L   I   E   N   S   P   S   V   W   Q   N   I   L   E   S   D   T   E   F

        3190      3200      3210      3220      3230      3240
AAAAAAGTGACACCTTTGATTCATGACAGAATGCTTATGGACAAAAATGCTACAGCTTTG
 K   K   V   T   P   L   I   H   D   R   M   L   M   D   K   N   A   T   A   L      1059

        3250      3260      3270      3280      3290      3300
AGGCTAAATCATATGTCAAATAAAACTACTTCATCAAAAAACATGGAAATGGTCCAACAG
 R   L   N   H   M   S   N   K   T   T   S   S   K   N   M   E   M   V   Q   Q

        3310      3320      3330      3340      3350      3360
AAAAAAGAGGGCCCCATTCCACCAGATGCACAAAATCCAGATATGTCGTTCTTTAAGATG
 K   K   E   G   P   I   P   P   D   A   Q   N   P   D   M   S   F   F   K   M      1099

        3370      3380      3390      3400      3410      3420
CTATTCTTGCCAGAATCAGCAAGGTGGATACAAAGGACTCATGGAAAGAACTCTCTGAAC
 L   F   L   P   E   S   A   R   W   I   Q   R   T   H   G   K   N   S   L   N

        3430      3440      3450      3460      3470      3480
TCTGGGCAAGGCCCCAGTCCAAAGCAATTAGTATCCTTAGGACCAGAAAAATCTGTGGAA
 S   G   Q   G   P   S   P   K   Q   L   V   S   L   G   P   E   K   S   V   E      1139

        3490      3500      3510      3520      3530      3540
GGTCAGAATTTCTTGTCTGAGAAAAACAAAGTGGTAGTAGGAAAGGGTGAATTTACAAAG
 G   Q   N   F   L   S   E   K   N   K   V   V   V   G   K   G   E   F   T   K

        3550      3560      3570      3580      3590      3600
GACGTAGGACTCAAAGAGATGGTTTTTCCAAGCAGCAGAAACCTATTTCTTACTAACTTG
 D   V   G   L   K   E   M   V   F   P   S   S   R   N   L   F   L   T   N   L      1179
```

```
          3610      3620      3630      3640      3650      3660
GATAATTTACATGAAAATAATACACACAATCAAGAAAAAAAAATTCAGGAAGAAATAGAA
 D   N   L   H   E   N   N   T   H   N   Q   E   K   K   I   Q   E   E   I   E

          3670      3680      3690      3700      3710      3720
AAGAAGGAAACATTAATCCAAGAGAATGTAGTTTTGCCTCAGATACATACAGTGACTGGC
 K   K   E   T   L   I   Q   E   N   V   V   L   P   Q   I   H   T   V   T   G      1219

          3730      3740      3750      3760      3770      3780
ACTAAGAATTTCATGAAGAACCTTTTCTTACTGAGCACTAGGCAAAATGTAGAAGGTTCA
 T   K   N   F   M   K   N   L   F   L   L   S   T   R   Q   N   V   E   G   S

          3790      3800      3810      3820      3830      3840
TATGACGGGGCATATGCTCCAGTACTTCAAGATTTTAGGTCATTAAATGATTCAACAAAT
 Y   D   G   A   Y   A   P   V   L   Q   D   F   R   S   L   N   D   S   T   N      1259

          3850      3860      3870      3880      3890      3900
AGAACAAAGAAACACACAGCTCATTTCTCAAAAAAAGGGGAGGAAGAAAACTTGGAAGGC
 R   T   K   K   H   T   A   H   F   S   K   K   G   E   E   N   L   E   G

          3910      3920      3930      3940      3950      3960
TTGGGAAATCAAACCAAGCAAATTGTAGAGAAATATGCATGCACCACAAGGATATCTCCT
 L   G   N   Q   T   K   Q   I   V   E   K   Y   A   C   T   T   R   I   S   P      1299

          3970      3980      3990      4000      4010      4020
AATACAAGCCAGCAGAATTTTGTCACGCAACGTAGTAAGAGAGCTTTGAAACAATTCAGA
 N   T   S   Q   Q   N   F   V   T   Q   R   S   K   R   A   L   K   Q   F   R

          4030      4040      4050      4060      4070      4080
CTCCCACTAGAAGAAACAGAACTTGAAAAAAGGATAATTGTGGATGACACCTCAACCCAG
 L   P   L   E   E   T   E   L   E   K   R   I   I   V   D   D   T   S   T   Q      1339

          4090      4100      4110      4120      4130      4140
TGGTCCAAAAACATGAAACATTTGACCCCGAGCACCCTCACACAGATAGACTACAATGAG
 W   S   K   N   M   K   H   L   T   P   S   T   L   T   Q   I   D   Y   N   E

          4150      4160      4170      4180      4190      4200
AAGGAGAAAGGGGCCATTACTCAGTCTCCCTTATCAGATTGCCTTACGAGGAGTCATAGC
 K   E   K   G   A   I   T   Q   S   P   L   S   D   C   L   T   R   S   H   S      1379

          4210      4220      4230      4240      4250      4260
ATCCCTCAAGCAAATAGATCTCCATTACCCATTGCAAAGGTATCATCATTTCCATCTATT
 I   P   Q   A   N   R   S   P   L   P   I   A   K   V   S   S   F   P   S   I

          4270      4280      4290      4300      4310      4320
AGACCTATATATCTGACCAGGGTCCTATTCCAAGACAACTCTTCTCATCTTCCAGCAGCA
 R   P   I   Y   L   T   R   V   L   F   Q   D   N   S   S   H   L   P   A   A      1419

          4330      4340      4350      4360      4370      4380
TCTTATAGAAAGAAAGATTCTGGGGTCCAAGAAAGCAGTCATTTCTTACAAGGAGCCAAA
 S   Y   R   K   K   D   S   G   V   Q   E   S   S   H   F   L   Q   G   A   K

          4390      4400      4410      4420      4430      4440
AAAAATAACCTTTCTTTAGCCATTCTAACCTTGGAGATGACTGGTGATCAAAGAGAGGTT
 K   N   N   L   S   L   A   I   L   T   L   E   M   T   G   D   Q   R   E   V      1459

          4450      4460      4470      4480      4490      4500
GGCTCCCTGGGGACAAGTGCCACAAATTCAGTCACATACAAGAAAGTTGAGAACACTGTT
 G   S   L   G   T   S   A   T   N   S   V   T   Y   K   K   V   E   N   T   V
```

```
          4510      4520      4530      4540      4550      4560
CTCCCGAAACCAGACTTGCCCAAAACATCTGGCAAAGTTGAATTGCTTCCAAAAGTTCAC
 L   P   K   P   D   L   P   K   T   S   G   K   V   E   L   L   P   K   V   H    1499

          4570      4580      4590      4600      4610      4620
ATTTATCAGAAGGACCTATTCCCTACGGAAACTAGCAATGGGTCTCCTGGCCATCTGGAT
 I   Y   Q   K   D   L   F   P   T   E   T   S   N   G   S   P   G   H   L   D

          4630      4640      4650      4660      4670      4680
CTCGTGGAAGGGAGCCTTCTTCAGGGAACAGAGGGAGCGATTAAGTGGAATGAAGCAAAC
 L   V   E   G   S   L   L   Q   G   T   E   G   A   I   K   W   N   E   A   N    1539

          4690      4700      4710      4720      4730      4740
AGACCTGGAAAAGTTCCCTTTCTGAGAGTAGCAACAGAAAGCTCTGCAAAGACTCCCTCC
 R   P   G   K   V   P   F   L   R   V   A   T   E   S   S   A   K   T   P   S

          4750      4760      4770      4780      4790      4800
AAGCTATTGGATCCTCTTGCTTGGGATAACCACTATGGTACTCAGATACCAAAAGAAGAG
 K   L   L   D   P   L   A   W   D   N   H   Y   G   T   Q   I   P   K   E   E    1579

          4810      4820      4830      4840      4850      4860
TGGAAATCCCAAGAGAAGTCACCAGAAAAAACAGCTTTTAAGAAAAAGGATACCATTTTG
 W   K   S   Q   E   K   S   P   E   K   T   A   F   K   K   K   D   T   I   L

          4870      4880      4890      4900      4910      4920
TCCCTGAACGCTTGTGAAAGCAATCATGCAATAGCAGCAATAAATGAGGGACAAAATAAG
 S   L   N   A   C   E   S   N   H   A   I   A   A   I   N   E   G   Q   N   K    1619

          4930      4940      4950      4960      4970      4980
CCCGAAATAGAAGTCACCTGGGCAAAGCAAGGTAGGACTGAAAGGCTGTGCTCTCAAAAC
 P   E   I   E   V   T   W   A   K   Q   G   R   T   E   R   L   C   S   Q   N
                                                            37  38

          4990      5000      5010      5020      5030      5040
CCACCAGTCTTGAAACGCCATCAACGGGAAATAACTCGTACTACTCTTCAGTCAGATCAA
 P   P   V   L   K   R   H   Q   R   E   I   T   R   T   T   L   Q   S   D   Q    1659
                             49

          5050      5060      5070      5080      5090      5100
GAGGAAATTGACTATGATGATACCATATCAGTTGAAATGAAGAAGGAAGATTTTGACATT
 E   E   I   D   Y   D   D   T   I   S   V   E   M   K   K   E   D   F   D   I

          5110      5120      5130      5140      5150      5160
TATGATGAGGATGAAAATCAGAGCCCCCGCAGCTTTCAAAAGAAAACACGACACTATTTT
 Y   D   E   D   E   N   Q   S   P   R   S   F   Q   K   K   T   R   H   Y   F    1699
                             89  90

          5170      5180      5190      5200      5210      5220
ATTGCTGCAGTGGAGAGGCTCTGGGATTATGGGATGAGTAGCTCCCCACATGTTCTAAGA
 I   A   A   V   E   R   L   W   D   Y   G   M   S   S   P   H   V   L   R

          5230      5240      5250      5260      5270      5280
AACAGGGCTCAGAGTGGCAGTGTCCCTCAGTTCAAGAAAGTTGTTTTCCAGGAATTTACT
 N   R   A   Q   S   G   S   V   P   Q   F   K   K   V   V   F   Q   E   F   T    1739

          5290      5300      5310      5320      5330      5340
GATGGCTCCTTTACTCAGCCCTTATACCGTGGAGAACTAAATGAACATTTGGGACTCCTG
 D   G   S   F   T   Q   P   L   Y   R   G   E   L   N   E   H   L   G   L   L

          5350      5360      5370      5380      5390      5400
GGGCCATATATAAGAGCAGAAGTTGAAGATAATATCATGGTAACTTTCAGAAATCAGGCC
 G   P   Y   I   R   A   E   V   E   D   N   I   M   V   T   F   R   N   Q   A    1779
```

```
        5410      5420      5430      5440      5450      5460
TCTCGTCCCTATTCCTTCTATTCTAGCCTTATTTCTTATGAGGAAGATCAGAGGCAAGGA
 S   R   P   Y   S   F   Y   S   S   L   I   S   Y   E   E   D   Q   R   Q   G

        5470      5480      5490      5500      5510      5520
GCAGAACCTAGAAAAAACTTTGTCAAGCCTAATGAAACCAAAACTTACTTTTGGAAAGTG
 A   E   P   R   K   N   F   V   K   P   N   E   T   K   T   Y   F   W   K   V      1819

        5530      5540      5550      5560      5570      5580
CAACATCATATGGCACCCACTAAAGATGAGTTTGACTGCAAAGCCTGGGCTTATTTCTCT
 Q   H   H   M   A   P   T   K   D   E   F   D   C   K   A   W   A   Y   F   S

        5590      5600      5610      5620      5630      5640
GATGTTGACCTGGAAAAAGATGTGCACTCAGGCCTGATTGGACCCCTTCTGGTCTGCCAC
 D   V   D   L   E   K   D   V   H   S   G   L   I   G   P   L   L   V   C   H      1859

        5650      5660      5670      5680      5690      5700
ACTAACACACTGAACCCTGCTCATGGGAGACAAGTGACAGTACAGGAATTTGCTCTGTTT
 T   N   T   L   N   P   A   H   G   R   Q   V   T   V   Q   E   F   A   L   F

        5710      5720      5730      5740      5750      5760
TTCACCATCTTTGATGAGACCAAAAGCTGGTACTTCACTGAAAATATGGAAAGAAACTGC
 F   T   I   F   D   E   T   K   S   W   Y   F   T   E   N   M   E   R   N   C      1899

        5770      5780      5790      5800      5810      5820
AGGGCTCCCTGCAATATCCAGATGGAAGATCCCACTTTTAAAGAGAATTATCGCTTCCAT
 R   A   P   C   N   I   Q   M   E   D   P   T   F   K   E   N   Y   R   F   H

        5830      5840      5850      5860      5870      5880
GCAATCAATGGCTACATAATGGATACACTACCTGGCTTAGTAATGGCTCAGGATCAAAGG
 A   I   N   G   Y   I   M   D   T   L   P   G   L   V   M   A   Q   D   Q   R      1939

        5890      5900      5910      5920      5930      5940
ATTCGATGGTATCTGCTCAGCATGGGCAGCAATGAAAACATCCATTCTATTCATTTCAGT
 I   R   W   Y   L   L   S   M   G   S   N   E   N   I   H   S   I   H   F   S

        5950      5960      5970      5980      5990      6000
GGACATGTGTTCACTGTACGAAAAAAAGAGGAGTATAAAATGGCACTGTACAATCTCTAT
 G   H   V   F   T   V   R   K   K   E   E   Y   K   M   A   L   Y   N   L   Y      1979

        6010      6020      6030      6040      6050      6060
CCAGGTGTTTTTGAGACAGTGGAAATGTTACCATCCAAAGCTGGAATTTGGCGGGTGGAA
 P   G   V   F   E   T   V   E   M   L   P   S   K   A   G   I   W   R   V   E

        6070      6080      6090      6100      6110      6120
TGCCTTATTGGCGAGCATCTACATGCTGGGATGAGCACACTTTTTCTGGTGTACAGCAAT
 C   L   I   G   E   H   L   H   A   G   M   S   T   L   F   L   V   Y   S   N      2019

        6130      6140      6150      6160      6170      6180
AAGTGTCAGACTCCCCTGGGAATGGCTTCTGGACACATTAGAGATTTTCAGATTACAGCT
 K   C   Q   T   P   L   G   M   A   S   G   H   I   R   D   F   Q   I   T   A

        6190      6200      6210      6220      6230      6240
TCAGGACAATATGGACAGTGGGCCCCAAAGCTGGCCAGACTTCATTATTCCGGATCAATC
 S   G   Q   Y   G   Q   W   A   P   K   L   A   R   L   H   Y   S   G   S   I      2059

        6250      6260      6270      6280      6290      6300
AATGCCTGGAGCACCAAGGAGCCCTTTTCTTGGATCAAGGTGGATCTGTTGGCACCAATG
 N   A   W   S   T   K   E   P   F   S   W   I   K   V   D   L   L   A   P   M
```

```
        6310      6320      6330      6340      6350      6360
ATTATTCACGGCATCAAGACCCAGGGTGCCCGTCAGAAGTTCTCCAGCCTCTACATCTCT
  I   I   H   G   I   K   T   Q   G   A   R   Q   K   F   S   S   L   Y   I   S    2099

        6370      6380      6390      6400      6410      6420
CAGTTTATCATCATGTATAGTCTTGATGGGAAGAAGTGGCAGACTTATCGAGGAAATTCC
  Q   F   I   I   M   Y   S   L   D   G   K   K   W   Q   T   Y   R   G   N   S

        6430      6440      6450      6460      6470      6480
ACTGGAACCTTAATGGTCTTCTTTGGCAATGTGGATTCATCTGGGATAAAACACAATATT
  T   G   T   L   M   V   F   F   G   N   V   D   S   S   G   I   K   H   N   I    2139

        6490      6500      6510      6520      6530      6540
TTTAACCCTCCAATTATTGCTCGATACATCCGTTTGCACCCAACTCATTATAGCATTCGC
  F   N   P   P   I   I   A   R   Y   I   R   L   H   P   T   H   Y   S   I   R

        6550      6560      6570      6580      6590      6600
AGCACTCTTCGCATGGAGTGGATGGGCTGTGATTTAAATAGTTGCAGCATGCCATTGGGA
  S   T   L   R   M   E   W   M   G   C   D   L   N   S   C   S   M   P   L   G    2179

        6610      6620      6630      6640      6650      6660
ATGGAGAGTAAAGCAATATCAGATGCACAGATTACTGCTTCATCCTACTTTACCAATATG
  M   E   S   K   A   I   S   D   A   Q   I   T   A   S   S   Y   F   T   N   M

        6670      6680      6690      6700      6710      6720
TTTGCCACCTGGTCTCCTTCAAAAGCTCGACTTCACCTCCAAGGGAGGAGTAATGCCTGG
  F   A   T   W   S   P   S   K   A   R   L   H   L   Q   G   R   S   N   A   W    2219

        6730      6740      6750      6760      6770      6780
AGACCTCAGGTGAATAATCCAAAAGAGTGGCTGCAAGTGGACTTCCAGAAGACAATGAAA
  R   P   Q   V   N   N   P   K   E   W   L   Q   V   D   F   Q   K   T   M   K

        6790      6800      6810      6820      6830      6840
GTCACAGGAGTAACTACTCAGGGAGTAAAATCTCTGCTTACCAGCATGTATGTGAAGGAG
  V   T   G   V   T   T   Q   G   V   K   S   L   L   T   S   M   Y   V   K   E    2259

        6850      6860      6870      6880      6890      6900
TTCCTCATCTCCAGCAGTCAAGATGGCCATCAGTGGACTCTCTTTTTTCAGAATGGCAAA
  F   L   I   S   S   S   Q   D   G   H   Q   W   T   L   F   F   Q   N   G   K

        6910      6920      6930      6940      6950      6960
GTAAAGGTTTTTCAGGGAAATCAAGACTCCTTCACACCTGTGGTGAACTCTCTAGACCCA
  V   K   V   F   Q   G   N   Q   D   S   F   T   P   V   V   N   S   L   D   P    2299

        6970      6980      6990      7000      7010      7020
CCGTTACTGACTCGCTACCTTCGAATTCACCCCCAGAGTTGGGTGCACCAGATTGCCCTG
  P   L   L   T   R   Y   L   R   I   H   P   Q   S   W   V   H   Q   I   A   L

        7030      7040      7050      7060      7070      7080
AGGATGGAGGTTCTGGGCTGCGAGGCACAGGACCTCTACTGAGGGTGGCCACTGCAGCAC
  R   M   E   V   L   G   C   E   A   Q   D   L   Y
                                          2332 stop
        7090      7100      7110      7120      7130      7140
CTGCCACTGCCGTCACCTCTCCCTCCTCAGCTCCAGGGCAGTGTCCCTCCCTGGCTTGCC


        7150      7160      7170      7180      7190      7200
TTCTACCTTTGTGCTAAATCCTAGCAGACACTGCCTTGAAGCCTCCTGAATTAACTATCA
```

```
      7210      7220      7230      7240      7250      7260
TCAGTCCTGCATTTCTTTGGTGGGGGGCCAGGAGGGTGCATCCAATTTAACTTAACTCTT


      7270      7280      7290      7300      7310      7320
ACCTATTTTCTGCAGCTGCTCCCAGATTACTCCTTCCTTCCAATATAACTAGGCAAAAAG


      7330      7340      7350      7360      7370      7380
AAGTGAGGAGAAACCTGCATGAAAGCATTCTTCCCTGAAAAGTTAGGCCTCTCAGAGTCA


      7390      7400      7410      7420      7430      7440
CCACTTCCTCTGTTGTAGAAAAACTATGTGATGAAACTTTGAAAAAGATATTTATGATGT


      7450      7460      7470      7480      7490      7500
TAACATTTCAGGTTAAGCCTCATACGTTTAAAATAAAACTCTCAGTTGTTTATTATCCTG


      7510      7520      7530      7540      7550      7560
ATCAAGCATGGAACAAAGCATGTTTCAGGATCAGATCAATACAATCTTGGAGTCAAAAGG


      7570      7580      7590      7600      7610      7620
CAAATCATTTGGACAATCTGCAAAATGGAGAGAATACAATAACTACTACAGTAAAGTCTG


      7630      7640      7650      7660      7670      7680
TTTCTGCTTCCTTACACATAGATATAATTATGTTATTTAGTCATTATGAGGGGCACATTC


      7690      7700      7710      7720      7730      7740
TTATCTCCAAAACTAGCATTCTTAAACTGAGAATTATAGATGGGGTTCAAGAATCCCTAA


      7750      7760      7770      7780      7790      7800
GTCCCCTGAAATTATATAAGGCATTCTGTATAAATGCAAATGTGCATTTTTCTGACGAGT


      7810      7820      7830      7840      7850      7860
GTCCATAGATAAAAAGCCATTTGGTCTTAATTCTGACCAATAAAAAAATAAGTCAGGAGG


      7870      7880      7890      7900      7910      7920
ATGCAATTGTTGAAAGCTTTGAAATAAAATAACAATGTCTTCTTGAAATTTGTGATGGCC


      7930      7940      7950      7960      7970      7980
AAGAAAGAAAATGATGATGACATTAGGCTTCTAAAGGACATACATTTAATATTTCTGTGG


      7990      8000      8010      8020      8030      8040
AAATATGAGGAAAATCCATGGTTATCTGAGATAGGAGATACAAACTTTGTAATTCTAATA


      8050      8060      8070      8080      8090      8100
ATGCACTCAGTTTACTCTCTCCCTCTACTAATTTCCTGCTGAAAATAACACAACAAAAAT
```

GTAACAGGGGAAATTATATACCGTGACTGAAAACTAGAGTCCTACTTACATAGTTGAAAT

ATCAAGGAGGTCAGAAGAAAATTGGACTGGTGAAAACAGAAAAAACACTCCAGTCTGCCA

TATCACCACACAATAGGATCC

0253455

REFERENCES

Anagnostopoulos, C. and Spizizen, J. (1961) J. Bacteriol. <u>81</u>, 741-746.

Andreoli, P.M. (1985) Mol. Gen. Genet. <u>199</u>, 372-380.

Ano, T. et al., (1986) Mol. Gen. Genet. <u>202</u>, 416-420.

Arraj, J.A. and Marinus, M.G., (1983) J. Bacteriol, <u>153</u>, 562-565

Aviv, H. and Leder, P. (1972) Proc. Natl. Acad. Sci. USA <u>69</u>, 1408-1412

Breunig, K.D. et al (1984) Nucl. Acids Res. <u>12</u>, 2327-2341.

Brinkhous, K.M. et al., (1985) Proc. Natl. Acad. Sci. USA <u>82</u>, 8752-8756

Chang, A.C.Y. and Cohen, S.N. (1978) J. Bacteriol. <u>134</u>, 1141-1156

Chang, S. and Cohen, S.N. (1979) Mol. Gen. Genet. <u>168</u>, 111-115.

Chargwin,J.M. et al., (1979)Biochemistry <u>18</u>, 5294-5299

Das, S. and Hollenberg, C.P. (1982) Curr. Genet. <u>6</u>, 123-128.

Eaton, D. et al., (1986) Biochemistry <u>25</u>, 505-512

Enger-Valk, B.E. Ph.D. thesis (1981), University of Wageningen, The Netherlands.

Fay, P.J. et al., (1986) Biochim. Biophys. Acta 871, 268-278

Foltman, B. (1970) Methods Enzymol. 19, 421-436

Galfré, G. et al.,(1977) Nature 266, 550-552

Gandhi, A.P. and Kjaergaard, L. (1975) Biotechnol. Bioeng. 17, 1109-1118.

Grunstein, M. and Hogness, D.S. (1975) Proc. Natl. Acad. Sci. USA 72 (1975) 3961-3965.

Gubler, U. and Hoffman, B.J. (1983) Gene 25, 263-269.
Hanahan, D. (1983) J, Mol.Biol. 166, 557-580.

Holmes, D.S. and Quigley, M. (1981) Anal. Biochem 114, 193-197.

Ito, H., Murata, K. and Kimura, A. (1984) Agric. Biol. Chem. 48, 341-347.

Kawamura, F. and Doi, R.H. (1984) J. Bacteriol. 160, 442-444

Laemli, U.K. (1970) Nature 227, 680-685.

Maniatis, T. et al.,  (1982). Molecular cloning. Cold Spring Harbor Laboratory.

Marinus, M.G. and Morris, N.R. (1973), J. Bacteriol. 114, 143-1150

Matsumura, M. et al., (1984) J. Bacteriol. 160, 413-420.

Maxam, A.M. and Gilbert, W. (1980) Methods Enzymol. 65, 499-560.

McMaster, G.K. and Carmichal, G.G. (1977) Proc. Natl. Acad. Sci. USA 74, 4835-4838.

Melton, D.A. et al (1984) Nucl. Acids. Res. 12, 7035-7056.

Messing, J. and Vieira, J. (1982) Gene 19, 269-276.

Meyer, H.P. and Fiechter, A. (1985) Appl. Environ. Microbiol. 50, 503-507.

Miller, J.H. (1972), Experiments in Molecular Genetics, Cold Spring, Harbor Laboratory, New York.
Niaudet, B. et al., (1984) Mol. Gen. Genet. 197, 46-54.

Ohmura, K. et al., (1984) J. Biochem. 95, 87-93.

Palva, I. et al., (1983) Gene 22, 229-235.

Polak, J. and Novick, R.P. (1982) Plasmid 7 152-162.

Sanger, F. et al. (1977) Proc. Natl. Acad. Sci USA 74, 5463-5467.

Sarvas, M. (1986), Current Topics in Microbiol. and Immunol. 103-125 H.C. Wu and P.C. Tai eds., Springer Verlag.

Shiroza, T. et al. (1985) Gene 34, 1-8.
Stel, H.V. (1984) Ph.D. Thesis, University of Amsterdam, The Netherlands.

Stel, H.V. et al., (1983) Nature 303, 530-533.

Thorne, C.B. and Stull, H.B. (1966) J. Bacteriol. 91, 1012-1020.
Toole, J.J. et al (1984) Nature 312, 342-347.

Towbin H. et al., (1979) Proc. Natl. Acad. Sci. USA 76, 4350-4354.

Truett, M.A. et al., (1985) DNA 4, 333-349.

Twigg, A.J. and Sherratt, D. (1980) Nature 283, 216-218.

Ulmanen, I. et al., (1985) J. Bacteriol. 162, 176-182.

Van Mourik J. A. and Mochtar, J.A. (1970) Biochim. Biophys. Acta 221, 677-679.

Vehar, G. et al. (1984) Nature 312, 337-342.

Wood, W.I. et al. (1984). Nature 312, 330-337.

Yanisch-Perron, C. et al., (1985) Gene 33, 103-119.

# CLAIMS

1. An expression cassette comprising in the direction of transcription, a transcriptional and translational initiation regulatory region, optionally a secretion signal sequence, an open reading frame encoding an amino acid sequence immunologically cross-reactive with factor VIII, and a translational and transcriptional termination regulatory region, said regulatory regions being functional in a microbial host.

2. An expression cassette according to claim 1, wherein said open reading frame substantially encodes at least one of the 80 kDA protein or the 92 kDa protein of factor VIII.

3. An expression cassette according to claim 2, wherein said open reading frame encodes the 80 kDA and 92 kDa proteins joined by other means than the natural polypeptide bridge of factor VIII.

4. A vector comprising a replication system functional in a microbial host and an expression cassette comprising in the direction of transcription, a transcriptional and translational initiation regulatory region, optionally a secretion signal sequence, an open reading frame encoding an amino acid sequence immunologically cross-reactive with factor VIII, and a translational and transcriptional termination regulatory region, said regulatory regions being functional in a microbial host.

5. A vector according to claim 4, wherein said vector further comprises a marker for selection in a microbial host.

6. A vector according to claim 4, wherein said

0253455

- 59 -

open reading frame substantially encodes at least one of the 80 kDa protein or the 92 kDa protein of factor VIII.

7. A vector according to claim 4, wherein said open reading frame substantially encodes the 80 kDA protein.

8. A vector according to claim 4, wherein said open reading frame substantially encodes the 92 kDa protein.

9. A vector according to claim 4, wherein said open reading frame encodes the 80 kDA and 92 kDa proteins joined by other means than the natural polypeptide bridge of factor VIII.

10. A vector according to claim 9, wherein said 80 kDA and 92 kDa proteins are in the natural order of factor VIII.

11. Plasmid pGB850

12. Plasmid pGB852

13. Plasmid pGB853

14. Plasmid pGB861

15. Plasmid pGB864

16. Plasmid pOL92

17. Plasmid pOL80

18. Plasmid pPROM92/80PB

19 Vector pOL5

20. The preparation of a microbial host, characterized in that a <u>Bacillus subtilis</u> or a <u>Bacillus licheniformis</u>, which is alpha-amylase deficient and/or exoprotease deficient is transformed with a vector according to any one of claims 4-10 or 16-18.

21. A transformed microbial host comprising an expression cassette according to any one of claims 1-3.

22. A microbial host according to claim 21, wherein said host is a bacterium or a yeast.

23. A microbial host according to claim 22, wherein said bacterium is a <u>Bacillus</u> and the yeast is a <u>Kluyveromyces</u>.

24. A microbial host according to claim 23, wherein said bacterium is <u>Bacillus subtilis</u> or <u>Bacillus licheniformis</u>.

25. A <u>Bacillus</u> host according to any one of claims 23-24 wherein said expression cassette is present on the plasmid pOL92 , pOL80 or pPROM92/80PB.

26. A microbial host according to claim 23, wherein said <u>Kluyveromyces</u> is <u>Kluyveromyces</u> <u>lactis</u>.

27. A <u>Kluyveromyces</u> host according to claim 23 or 26, wherein said expression cassette is present on the plasmid pGB850, pGB852, pGB853, pGB861 or pGB864.

28. A process for producing a polypeptide in isolatable form cross-reactive with factor VIII, said process comprising;

cultivating a microbial host according to any of claims 21-27 in a nutrient medium to produce said polypeptide; and

isolating said polypeptide.

29. A process according to claim 28, wherein said open reading frame is joined at its 5' terminus to a DNA sequence encoding a signal sequence functional in said microbial host for secretion of said polypeptide and wherein said signal sequence is processed.

30. A process according to claim 29, wherein said microbial host is a Kluyveromyces.

31. A process according to claim 29, wherein said microbial host is a Bacillus.

32. A pharmaceutical composition comprising a polypeptide produced by the process comprising:
        cultivating a microbial host according to any of
        claims 21-27 in a nutrient medium to produce said
        polypeptide;  and
        isolating said polypeptide,
and a pharmaceutically acceptable carrier or diluent and optionally an adjuvant.

33. A polypeptide produced by the process comprising:
cultivating a microbial host according to any of claims 21-27 in a nutrient medium to produce said polypeptide, and isolating said polypeptide.

34. An antibody to a protein obtained by a process according to any one of claims 28-31.

35. A monoclonal antibody according to claim 34.

36. A method of purifying protein having factor VIII activity which comprises bringing a liquid phase comprising protein and a content of impurities into contact with a solid phase comprising antibody according to claim 34 or 35, separating the liquid phase from the solid phase and eluting from the solid phase protein with a reduced content of impurities.

37. A method of diagnosing a blood sample for factor VIII abnormalities which comprises bringing the sample into contact with an antibody according to claim 34 or 35 and assaying for the presence or absence of a conjugate of the antibody with factor VIII antigen as a measure of the factor VIII protein in the sample.

Fig. 1

Fig. 2

m.c.s. EcoRI    XmaⅢ    XmaI    SalI    HindⅢ

5'pGAA TTC GCG GCC GCC CGG GTC GAC TCA AGC TTA AAT

TCG CGG CCG CCC GGG TCG ACT CAA GCT TAA ATT

CGC GGC CGC CCG GGT CGA CTC AAG CTT AAA 3'

Fig. 3

Fig. 4

4/27

0253455

Fig 5A

Fig. 5 B

Fig.5 C

7/27

0253455

pOL92-T-21
8.1kb

pOL92-T-21Δ
7.4kb

cut BglI | EcoRV

ligation

Fig. 6

← 94 kd

← 67 kd

← 43 kd

Fig. 7

0253455

Fig. 8

Fig. 9

Fig. 10 A B

A

B

13/27

0253455

C

Fig. 10 C D

D

Fig. 11

Fig. 12

deletion

ATG

TAA

ClaI

XbaI

SalI

pGBdLAC
7.6 kb

XbaI

Fig. 13

Fig. 14

Fig. 15

ATG
ClaI/BamHI
SacII
TGA
pGB852
12.6 kb
ClaI/HpaI
EcoRV
XbaI
SalI
XbaI

pGB853
11.8 kb.

pGB861
15.0 kb.

SalI
ATG
SacII
XbaI
PstI
TGA
ClaI/HpaI
EcoRV

Fig. 47

0253455

21/27

Fig. 18 A B

Fig. 19 AB

0253455

23/27

Fig. 19 C

0253455

24/27

Fig. 19 D

D

Fig. 19 E

1   2   3   4   5   6

E

0253455

26/27

Fig 19 F G H

0253455

27/27

Fig 19 I

0253455

**European Patent Office**

## EUROPEAN SEARCH REPORT

Application number

| | | | |
|---|---|---|---|
| **DOCUMENTS CONSIDERED TO BE RELEVANT** | | | EP 87201379.2 |

| Category | Citation of document with indication, where appropriate, of relevant passages | Relevant to claim | CLASSIFICATION OF THE APPLICATION (Int. Cl.4) |
|---|---|---|---|
| D,A | EP - A2 - 0 150 735 (CHIRON CORPO-RATION)<br><br>   * Pages 13,14; claims 1,4 *<br><br>-- | 1-10,<br>20-24,<br>34,35 | C 12 N 15/00<br>C 12 N 1/20<br>C 12 N 1/16<br>C 12 P 21/00<br>A 61 K 37/02 |
| D,A | EP - A1 - 0 160 457 (GENENTECH, INC.)<br>   * Claims *<br><br>-- | 1,4 | C 07 K 13/00<br>A 61 K 39/395<br>G 01 N 33/68<br>G 01 N 33/577<br>/(C 12 N 1/20 |
| D,A | DNA, vol. 4, no. 1, February 1985, New York<br><br>M.A.TRUETT et al. "Characterization of the Polypeptide Composition of Human Factor VIII:C and the Nucleotide Sequence and Expression of the Human Kidney cDNA"<br>pages 333-349<br><br>   * Pages 333-335 *<br><br>-- | 36,37 | C 12 R 1:125)<br>(C 12 N 1/20<br>C 12 R 1:10)<br>(C 12 N 1/20<br>C 12 R 1:07) |
| D,A | BIOCHEMICA ET BIOPHYSICA ACTA, vol. 871, June 23, 1986, Amsterdam<br><br>P.J.FAY et al. "The size of human factor VIII heterodimers and the effects produced by thrombin"<br>pages 268-278<br><br>   * Totality *<br><br>-- | | |

| | | |
|---|---|---|
| | | **TECHNICAL FIELDS SEARCHED (Int. Cl.4)**<br><br>C 12 N<br>C 12 P<br>A 61 K<br>C 07 K<br>G 01 N |

The present search report has been drawn up for all claims

| Place of search | Date of completion of the search | Examiner |
|---|---|---|
| VIENNA | 30-09-1987 | WOLF |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82

0253455

**European Patent Office**

## EUROPEAN SEARCH REPORT

Application number

| | DOCUMENTS CONSIDERED TO BE RELEVANT | | EP 87201379.2 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,A | BIOCHEMISTRY, vol. 25, no. 2, January 28, 1986, New York D.EATON et al. "Proteolytic Processing of Human Factor VIII. Correlation of Specific Cleavages by Thrombin, Factor Xa, and Activated Protein C with Activation and Inactivation of Factor VIII Coagulant Activity" pages 505-512 * Totality * ---- | | |
| | | | **TECHNICAL FIELDS SEARCHED (Int. Cl.4)** |

The present search report has been drawn up for all claims

| Place of search | Date of completion of the search | Examiner |
|---|---|---|
| VIENNA | 30-09-1987 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03.82

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

Accession numbers of the deposits: 
CBS 304.86  
CBS 303.86  
CBS 305.86  
CBS 306.86

EPO Form 1165 07.81 e